# EUROPEAN PATENT SPECIFICATION

(11) **EP 2 483 674 B1**
(45) Date of publication and mention of the grant of the patent: **27.11.2024**
(21) Application number: 10771238.2
(22) Date of filing: 01.10.2010
(51) Int. Cl.: G01N 27/49, G01N 33/487, C12Q 1/00, C08L 33/08

(54) **ANALYTE SENSOR APPARATUSES HAVING INTERFERENCE REJECTION MEMBRANES AND METHODS FOR MAKING AND USING THEM**
ANALYTSENSORGERÄTE MIT INTERFERENZABWEISUNGSMEMBRANEN SOWIE VERFAHREN ZU IHRER HERSTELLUNG UND VERWENDUNG
APPAREILS DE DÉTECTION D'ANALYTES COMPORTANT DES MEMBRANES DE REJET D'INTERFÉRENCES, ET PROCÉDÉS DE FABRICATION ET D'UTILISATION DE CEUX-CI

(30) Priority: 01.10.2009 US 572087
(43) Date of publication of application: 08.08.2012
(73) Proprietor: Medtronic MiniMed, Inc., Northridge, CA 91325-1219 (US)
(72) Inventor: QINGLING, Yang, Northbridge CA 91325 (US); SHAH, Rajiv, Rancho Palos Verdes CA 90275 (US); LITTLE, Megan E., Claremont CA 91711 (US); CHIU, Chia-Hung, Granada Hills CA 93144 (US)
(74) Representative: Zimmermann & Partner Patentanwälte mbB
(86) International application number: PCT/US2010/051177
(87) International publication number: WO 2011/041715

(56) References cited:
- WO-A1-98/38906
- WO-A2-2004/060297
- WO-A2-2005/012873
- WO-A2-2006/110193
- US-A1- 2007 129 697
- US-A1- 2010 121 169

## Description

### Background of the Invention

### 1. Field of the Invention.

Analyte sensors (e.g. glucose sensors used in the management of diabetes) and methods and materials for making and using such sensors.

### 2. Description of Related Art.

Analyte sensors such as biosensors include devices that use biological elements to convert a chemical analyte in a matrix into a detectable signal. There are many types of biosensors used for a wide variety of analytes. The most studied type of biosensor is the amperometric glucose sensor, which is crucial to the successful glucose level control for diabetes.

A typical glucose sensor works according to the following chemical reactions: 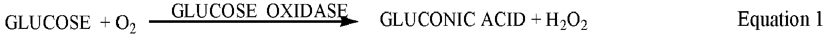 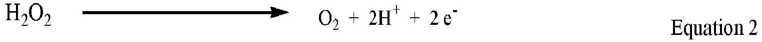 The glucose oxidase is used to catalyze the reaction between glucose and oxygen to yield gluconic acid and hydrogen peroxide (equation 1). The H₂O₂ reacts electrochemically as shown in equation 2, and the current can be measured by a potentiostat. These reactions, which occur in a variety of oxidoreductases known in the art, are used in a number of sensor designs.

One common problem with electrochemical sensors is that they can electrochemically react not only with the analyte to be measured (or by-product of the enzymatic reaction with the analyte), but additionally can react with other electroactive chemical species that are not intentionally being measured reaction which causes an increase in signal strength due to these "interfering species". Typically, such interfering species are compounds with an oxidation or reduction potential that overlaps with the analyte to be measured (or by product of the enzymatic reaction with the analyte). For example, in a conventional amperometric glucose oxidase-based glucose sensor wherein the sensor measures hydrogen peroxide, interfering species such as acetaminophen, ascorbate, and urate, are known to confound true analyte signals. For this reason, methods and materials designed to address difficulties caused by such interfering species are desirable.

WO 2005/012873 discloses systems and methods for electrochemical measurement of analytes. WO 98/38906 also discloses devices and methods for determining analyte levels. The devices and methods allow for the implantation of analyte-monitoring devices, such as glucose monitoring devices, that result in the delivery of a dependable flow of blood to the implanted device. WO 2006/110193 discloses an amperometric sensor with a cellulose interference rejection membrane.

### Summary of the Invention

The invention provides an amperometric analyte sensor apparatus comprising: a base layer; a conductive layer disposed on the base layer and comprising a working electrode; an interference rejection membrane (IRM) disposed on an electroactive surface of the working electrode, wherein the interference rejection membrane comprises crosslinked primary amine polymers or crosslinked methacrylate polymers. In illustrative embodiments of this sensor, the interference rejection membrane can comprise crosslinked methacrylate (e.g. Poly(2-hydroxyethyl methacrylate)) polymers having an average molecular weight between 100 and 1000 kilodaltons; and/or crosslinked primary amine polymers having an average molecular weight between 4 and 500 kilodaltons. Suitable primary amine polymers include polylysine polymers; poly(allylamine) polymers; amine terminated polyethylene oxide) polymers; poly(vinylamine) polymers; polyethylenimine polymers and the like. Suitable hydrophilic crosslinking compounds include urea and a hydrophilic organofunctional dipodal alkoxysilanes (e.g. to link methacrylate polymers) or glutaraldehyde (e.g. to link primary amine polymers). In certain embodiments of the invention, the interference rejection membrane is between 0.1 µm and 0.2 µm thick and is adhered to and in direct contact with an electrochemically reactive surface of a working electrode.

Typically, the interference rejection membrane inhibits the diffusion therethrough of compounds having a molecular weight greater than 140 Daltons (e.g. acetaminophen, uric acid, ascorbic acid and the like). In one such embodiment of the invention, the interference rejection membrane inhibits the diffusion of acetaminophen (molecular weight 151.17 Daltons) therethrough in a manner that decreases a signal in the analyte sensor apparatus that results from a concentration of acetaminophen by at least 50% as compared to a control analyte sensor apparatus lacking the interference rejection membrane.

Certain analyte sensor embodiments of the invention can be implanted in vivo and typically comprise a plurality of functional layers disposed on one another, for example a base layer; a conductive layer disposed on the base layer and comprising a working electrode; an interference rejection membrane disposed on an electroactive surface of the working electrode, an analyte sensing layer coated directly on top of the interference rejection membrane and further comprising at least one of: a protein layer disposed on the analyte sensing layer; an analyte modulating layer disposed on the analyte sensing layer or the protein layer, wherein the analyte modulating layer comprises a composition that modulates the diffusion of an analyte diffusing through the analyte modulating layer; an adhesion promoting layer disposed on the analyte sensing layer, wherein the adhesion promoting layer promotes the adhesion between the analyte sensing layer and an analyte modulating layer; or a cover layer disposed on the analyte sensor apparatus, wherein the cover layer comprises an aperture positioned on the cover layer so as to facilitate an analyte present in vivo accessing and diffusing through an analyte modulating layer; and then accessing the analyte sensing layer. Typically, the analyte sensing layer comprises an oxidoreductase that generates hydrogen peroxide upon exposure to a substrate for the oxidoreductase, wherein the amount of hydrogen peroxide generated by the oxidoreductase is proportional to the amount of substrate exposed to the oxidoreductase.

Certain analyte sensor embodiments of the invention include a conductive layer that comprises a plurality of electrodes including the working electrode, a counter electrode and a reference electrode. Optionally, the conductive layer comprises a plurality of working electrodes, counter electrodes and reference electrodes grouped together as a unit and positionally distributed on the conductive layer in a repeating pattern of units. In some embodiments of the invention, the sensor is operatively coupled to: a sensor input capable of receiving a signal from the sensor that is based on a sensed physiological characteristic value in vivo; and a processor coupled to the sensor input, wherein the processor is capable of characterizing one or more signals received from the sensor. Optionally, a pulsed voltage is used to observe a signal from a working electrode of a sensor.

Other objects, features and advantages of the present invention will become apparent to those skilled in the art from the following detailed description. It is to be understood, however, that the detailed description and specific examples, while indicating some embodiments of the present invention are given by way of illustration and not limitation..

### Brief Description of the Figures

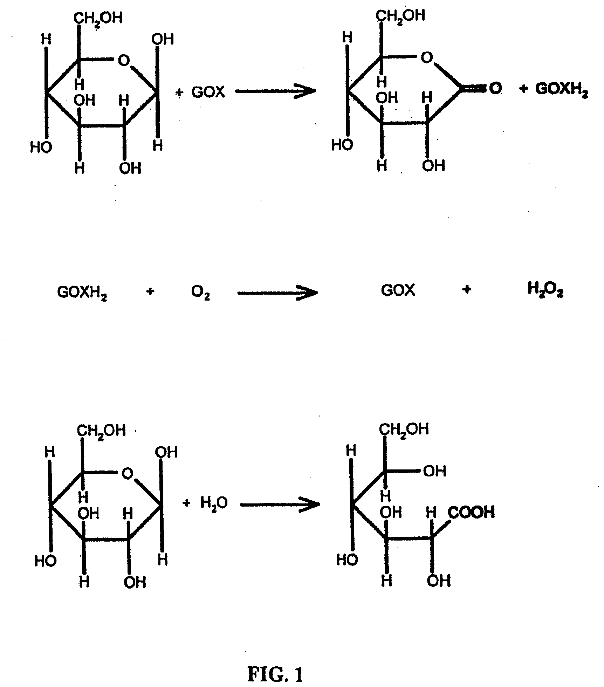

FIG. 1 provides a schematic of the well known reaction between glucose and glucose oxidase. As shown in a stepwise manner, this reaction involves glucose oxidase (GOx), glucose and oxygen in water. In the reductive half of the reaction, two protons and electrons are transferred from β-D-glucose to the enzyme yielding d-gluconolactone. In the oxidative half of the reaction, the enzyme is oxidized by molecular oxygen yielding hydrogen peroxide. The d-gluconolactone then reacts with water to hydrolyze the lactone ring and produce gluconic acid. In certain electrochemical sensors of the invention, the hydrogen peroxide produced by this reaction is oxidized at the working electrode (H₂O₂ → 2H+ + O₂ + 2e⁻).

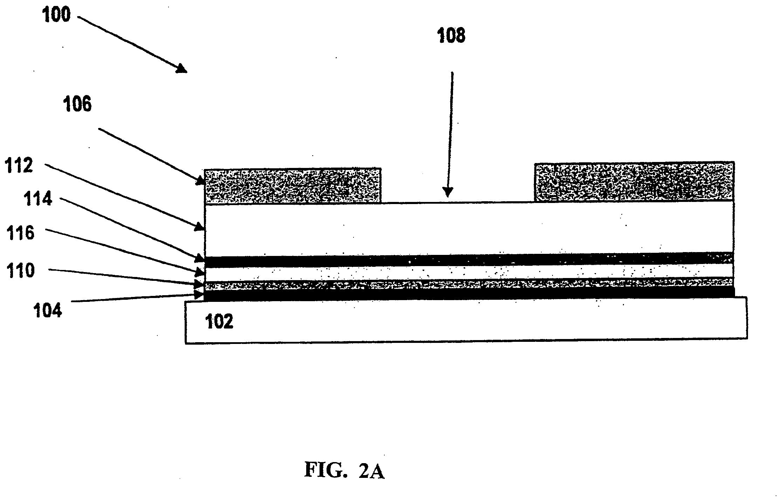

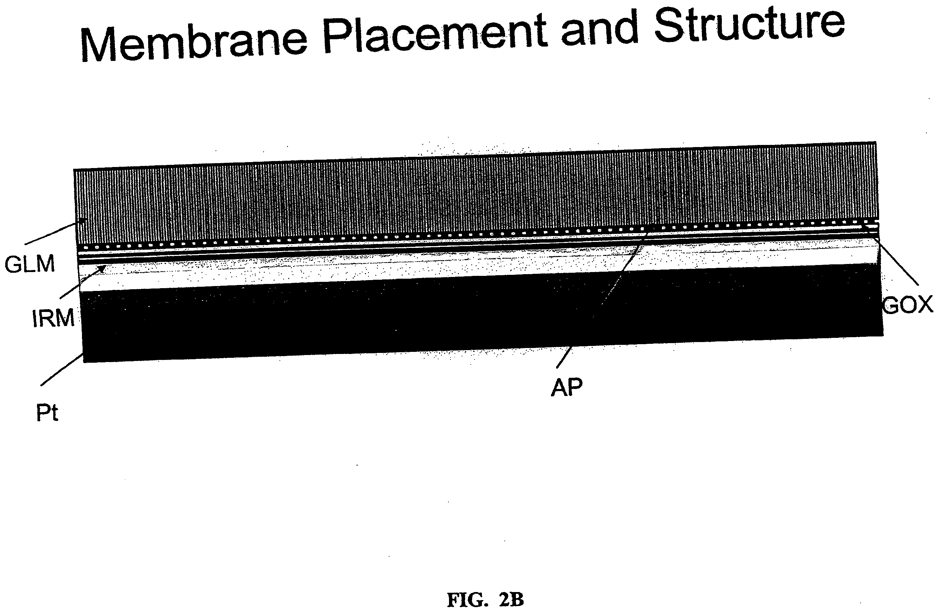

FIG. 2A provides a diagrammatic view of an amperometric analyte sensor to which an interference rejection membrane can be added. FIG. 2B provides a diagrammatic view of one embodiment of an amperometric analyte sensor having an interference rejection membrane.

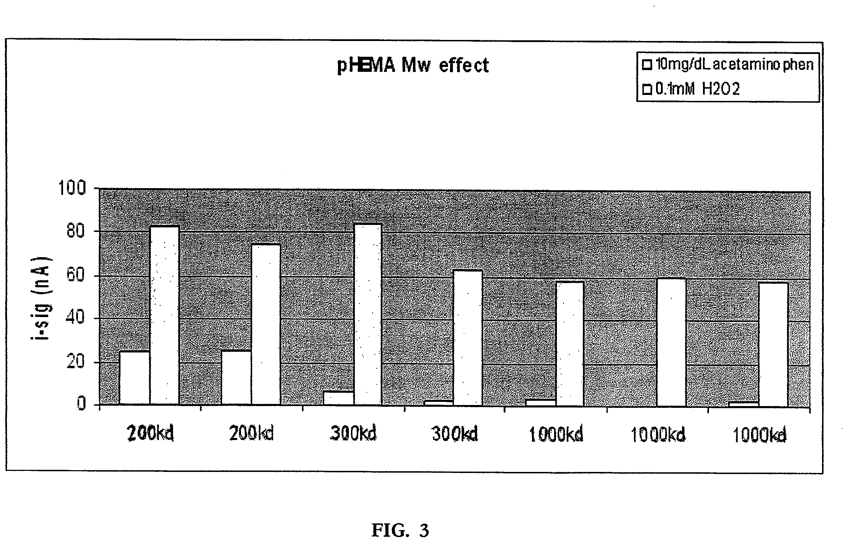

FIG. 3 provides data in the form of a bar graph which shows signals generated by acetaminophen and hydrogen peroxide in sensors having interference rejection membranes made from pHEMA polymers of differing molecular weights.

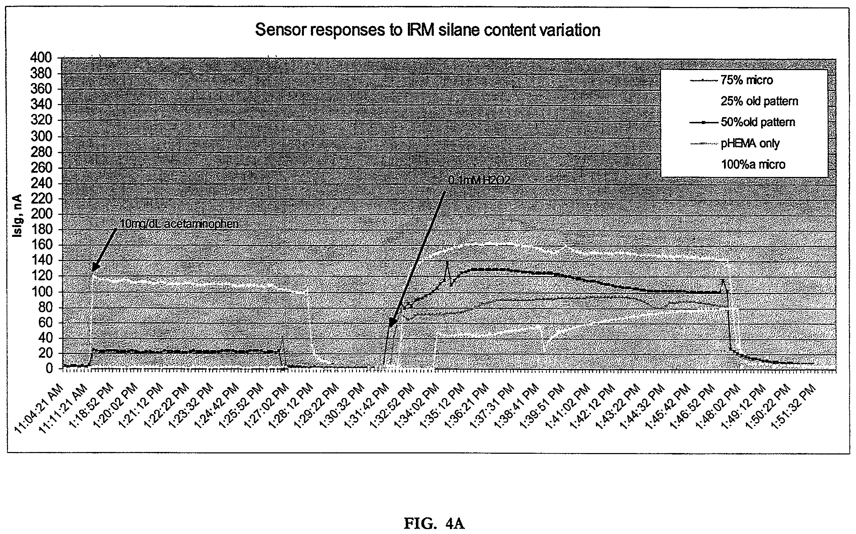

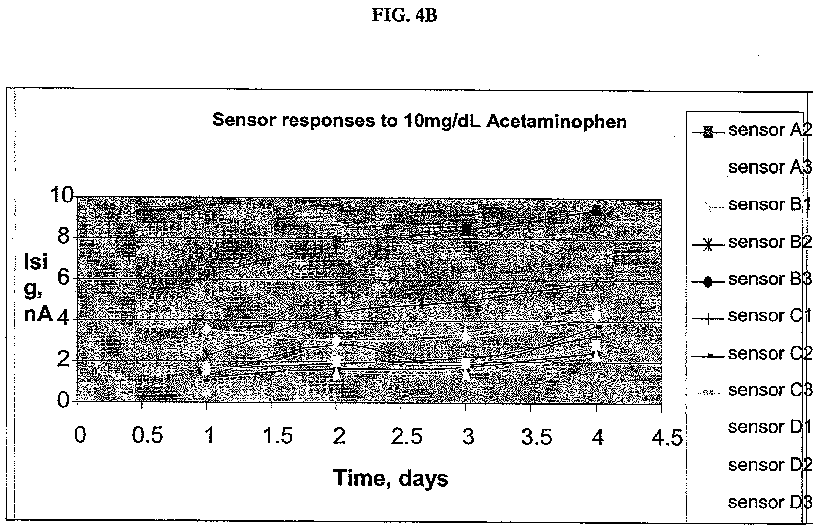

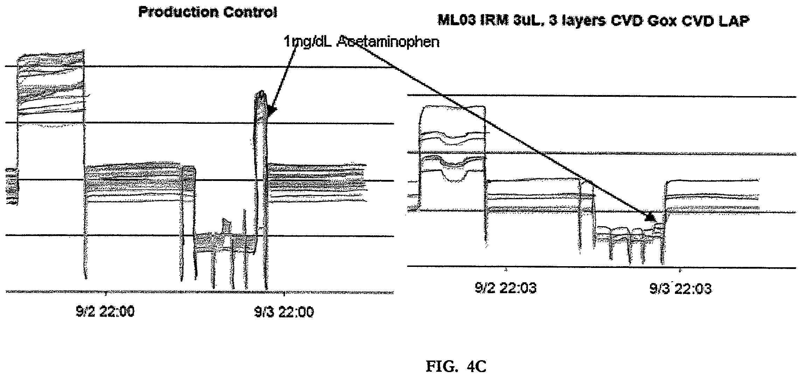

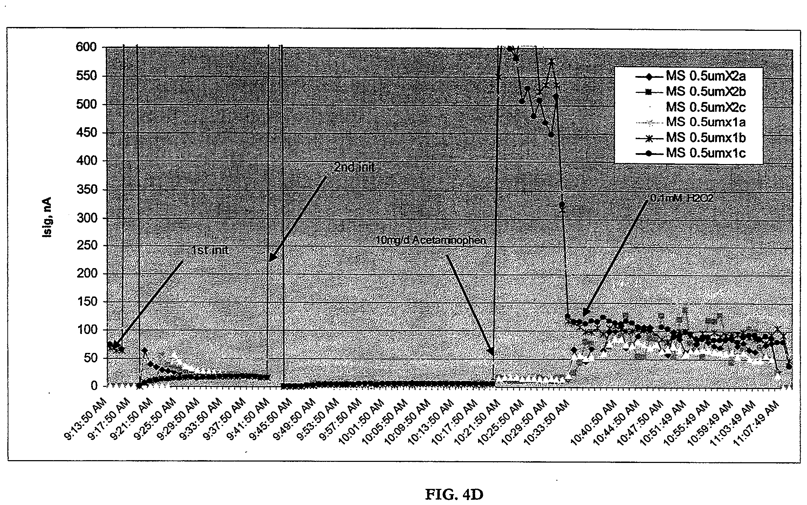

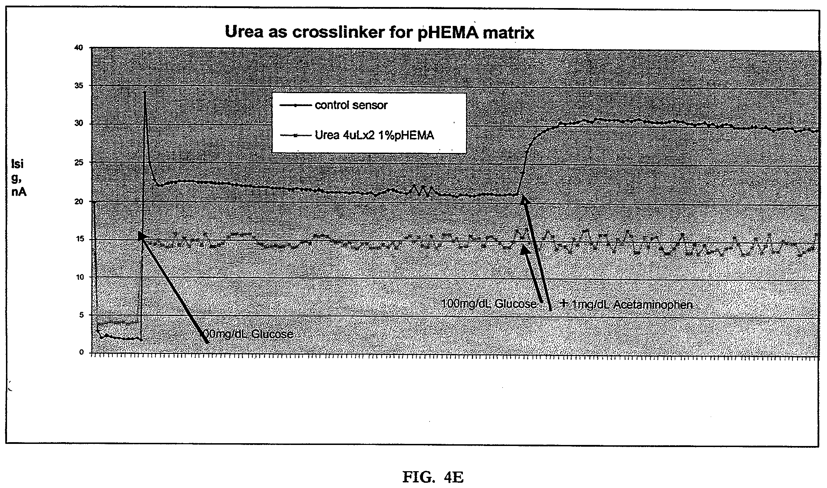

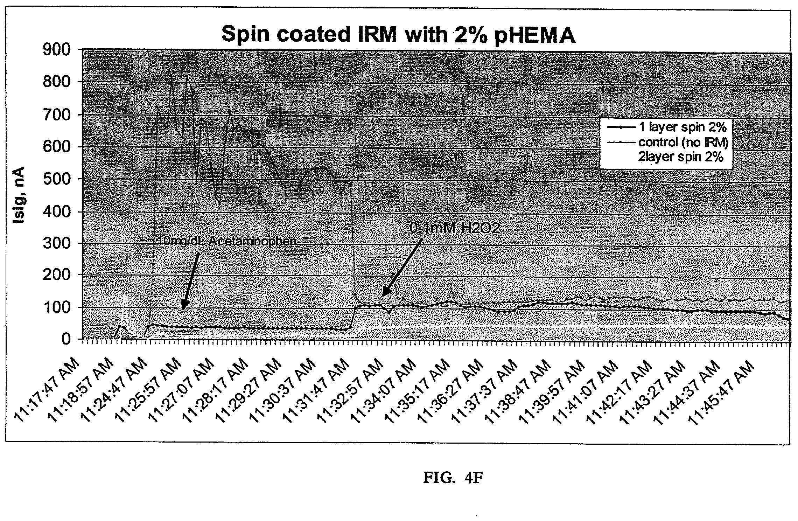

FIG. 4A provides graphic data showing sensor signals (Isig, nA) generated in response to acetaminophen and hydrogen peroxide and which shows the responses of different sensors, ones having IRMs crosslinked by differing amounts of a silane crosslinker (resulting in a different silane content in the crosslinked composition). FIG. 4B shows sensor responses to a dose of acetaminophen over periods of time. FIG. 4C shows IRM sensor interference rejection membrane performance in comparison with control sensors lacking such membranes. FIG. 4D shows data from a sensor with slotcoated IRM (sp2) responses to interfering compounds and H₂O₂. FIG. 4E shows data observing IRM sensor interference rejection membrane performance in comparison with control sensors lacking such membranes. FIG. 4F shows data observing IRM sensor interference rejection membrane performance (sensor coated with 1 or 2 layers of IRM composition) in comparison with control sensors lacking such membranes.

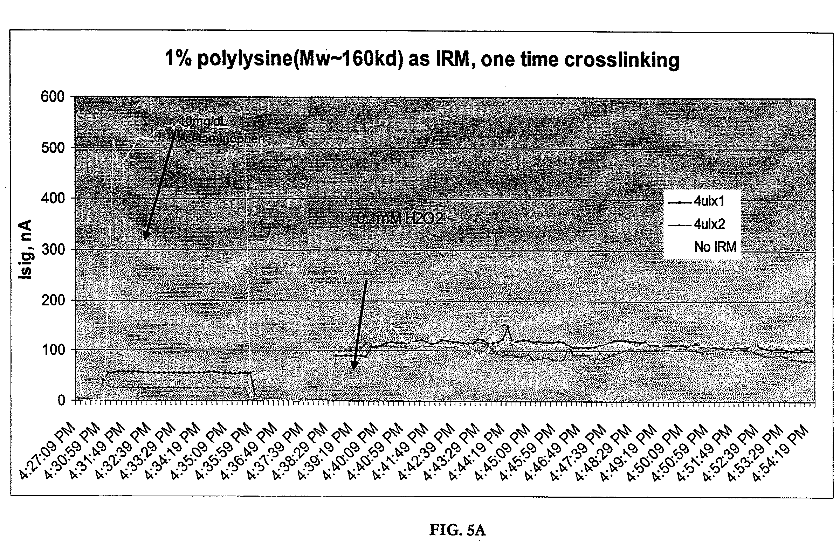

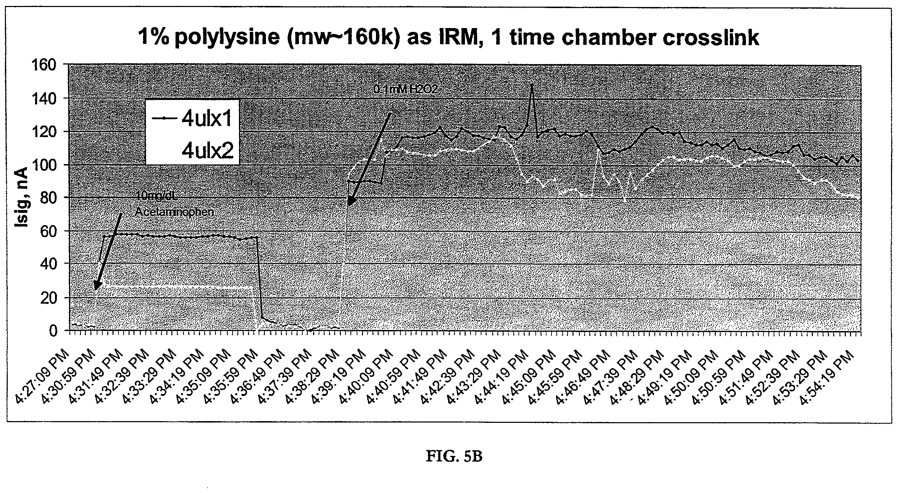

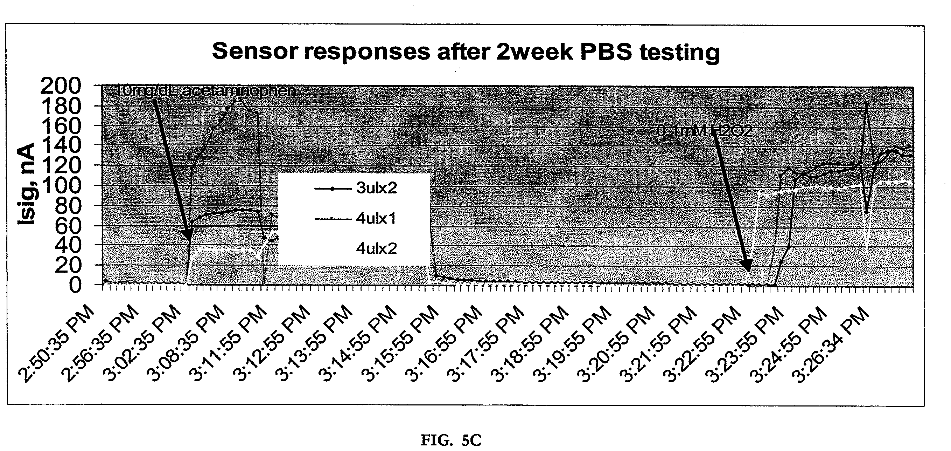

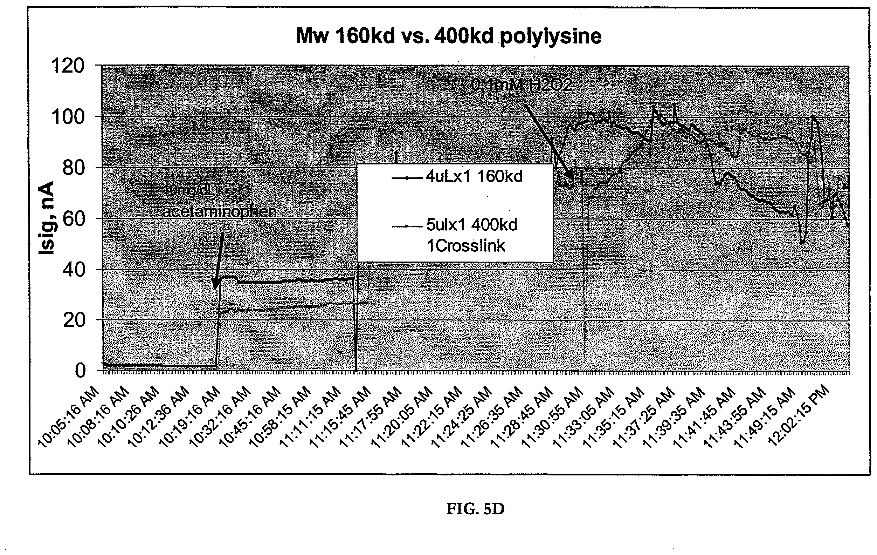

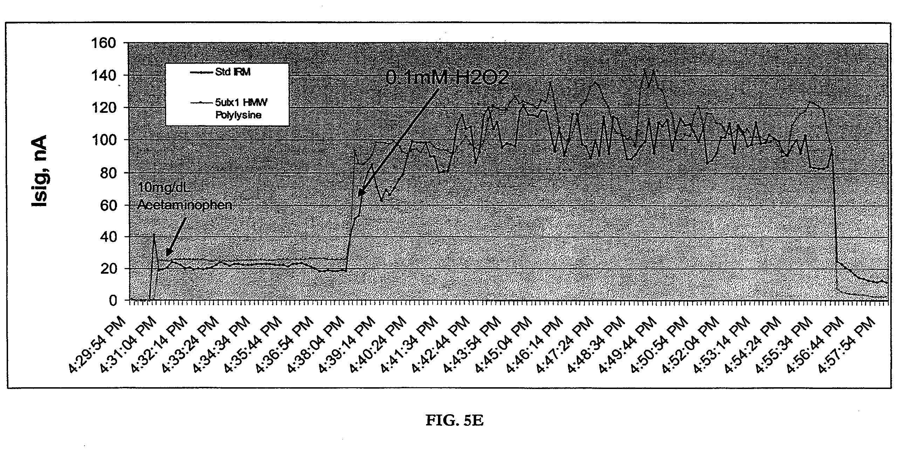

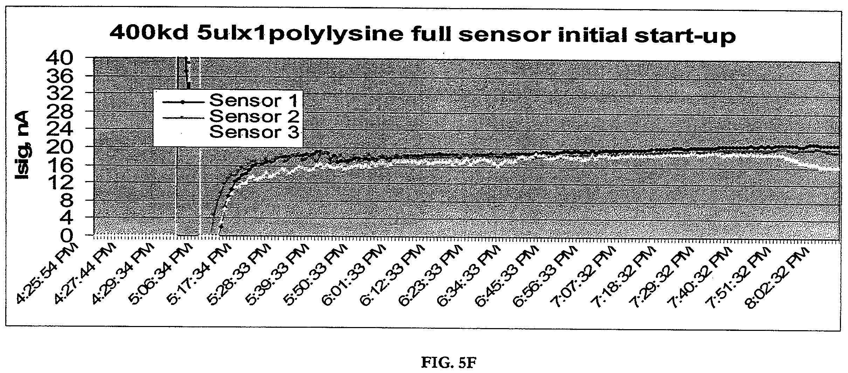

FIGS. 5A and 5B show sensor Interference Rejection Performance of IRM with 1% polylysine single crosslinking. This IRM efficiently inhibited interfering signals using a single layer applied via spraying. Two layers of a crosslinked polymer coating provide an interference rejection membrane with properties equivalent to pHEMA-silane systems disclosed in Examples 1 and 2. H₂O₂ ISig is not significantly decreased with IRM coatings comprising 2 layers of spray applied crosslinked polymer. Figure 5C shows IRM performances after 2 week of PBS testing and differences that can result from different process parameters, 4ul x2 shows stable interference capability and good H₂O₂. Figure 5D shows IRM performances from different molecular weight polylysine polymers. Figure 5E shows the performance of polylysine and pHEMA-silane IRM compositions. Figure 5F shows IRM sensor start-up performances during an initial 24 hrs, ones where the sensors started up within 30 minutes and did not show significant drift.

### Detailed Description of the Embodiments

Unless otherwise defined, all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art. Many of the techniques and procedures described or referenced herein are well understood and commonly employed using conventional methodology by those skilled in the art. As appropriate, procedures involving the use of commercially available kits and reagents are generally carried out in accordance with manufacturer defined protocols and/or parameters unless otherwise noted. A number of terms are defined below. Publications cited herein are cited for their disclosure prior to the filing date of the present application. Nothing here is to be construed as an admission that the inventors are not entitled to antedate the publications by virtue of an earlier priority date or prior date of invention. Further the actual publication dates may be different from those shown and require independent verification.

It must be noted that as used herein and in the appended claims, the singular forms "a", "and", and "the" include plural referents unless the context clearly dictates otherwise. Thus, for example, reference to "an oxidoreductase" includes a plurality of such oxidoreductases and equivalents thereof known to those skilled in the art, and so forth. All numbers recited in the specification and associated claims that refer to values that can be numerically characterized with a value other than a whole number (e.g. the concentration of a compound in a solution) are understood to be modified by the term "about".

The term "oxidoreductase" is used according to its art accepted meaning, i.e. an enzyme that catalyzes the transfer of electrons from one molecule (the reductant, also called the hydrogen or electron donor) to another (the oxidant, also called the hydrogen or electron acceptor). Typical oxidoreductases include glucose oxidase and lactate oxidase. The term "carrier polypeptide" or "carrier protein" is used according to its art accepted meaning of an additive included to maintain the stability of a polypeptide, for example the ability of an oxidoreductase polypeptide to maintain certain qualitative features such as physical and chemical properties (e.g. an ability to oxidize glucose) of a composition comprising a polypeptide for a period of time. A typical carrier protein commonly used in the art is albumin.

The term "analyte" as used herein is a broad term and is used in its ordinary sense, including, without limitation, to refer to a substance or chemical constituent in a fluid such as a biological fluid (for example, blood, interstitial fluid, cerebral spinal fluid, lymph fluid or urine) that can be analyzed. Analytes can include naturally occurring substances, artificial substances, metabolites, and/or reaction products. In some embodiments, the analyte for measurement by the sensing regions, devices, and methods is glucose. However, other analytes are contemplated as well, including but not limited to, lactate. Salts, sugars, proteins fats, vitamins and hormones naturally occurring in blood or interstitial fluids can constitute analytes in certain embodiments. The analyte can be naturally present in the biological fluid or endogenous; for example, a metabolic product, a hormone, an antigen, an antibody, and the like. Alternatively, the analyte can be introduced into the body or exogenous, for example, a contrast agent for imaging, a radioisotope, a chemical agent, a fluorocarbon-based synthetic blood, or a drug or pharmaceutical composition, including but not limited to insulin. The metabolic products of drugs and pharmaceutical compositions are also contemplated analytes.

The terms "interferents" and "interfering species/compounds" are used in their ordinary sense, including, but not limited to, effects and/or chemical species/compounds that interfere with the measurement of an analyte of interest in a sensor to produce a signal that does not accurately represent the analyte measurement. In one example of an electrochemical sensor, interfering species are compounds with an oxidation potential that overlaps with the analyte to be measured so as to produce spurious signals.

The term "sensor," as used herein, is a broad term and is used in its ordinary sense, including, without limitation, the portion or portions of an analyte-monitoring device that detects an analyte. In one embodiment, the sensor includes an electrochemical cell that has a working electrode, a reference electrode, and optionally a counter electrode passing through and secured within the sensor body forming an electrochemically reactive surface at one location on the body, an electronic connection at another location on the body, and a membrane system affixed to the body and covering the electrochemically reactive surface. During general operation of the sensor, a biological sample (for example, blood or interstitial fluid), or a portion thereof, contacts (directly or after passage through one or more membranes or domains) an enzyme (for example, glucose oxidase); the reaction of the biological sample (or portion thereof) results in the formation of reaction products that allow a determination of the analyte level in the biological sample.

The terms "electrochemically reactive surface" and "electroactive surface" as used herein are broad terms and are used in their ordinary sense, including, without limitation, the surface of an electrode where an electrochemical reaction takes place. In one example, a working electrode (e.g. one comprised of platinum black) measures hydrogen peroxide produced by the enzyme catalyzed reaction of the analyte being detected reacts creating an electric current (for example, detection of glucose analyte utilizing glucose oxidase produces H₂O₂ as a by product, H₂O₂ reacts with the surface of the working electrode producing two protons (2H⁺), two electrons (2e⁻) and one molecule of oxygen (O₂) which produces the electronic current being detected). In the case of the counter electrode, a reducible species, for example, O₂ is reduced at the electrode surface in order to balance the current being generated by the working electrode.

The term "sensing region" as used herein is a broad term and is used in its ordinary sense, including, without limitation, the region of a monitoring device responsible for the detection of a particular analyte. In an illustrative embodiment, the sensing region can comprise a non-conductive body, a working electrode, a reference electrode, and a counter electrode passing through and secured within the body forming electrochemically reactive surfaces on the body and an electronic connective means at another location on the body, and a one or more layers covering the electrochemically reactive surface.

As discussed in detail below, embodiments of the invention relate to the use of an electrochemical sensor that exhibits a novel constellation of elements including an interference rejection membrane having a unique set of technically desirable material properties. The electrochemical sensors of the invention are designed to measure a concentration of an analyte of interest (e.g. glucose) or a substance indicative of the concentration or presence of the analyte in fluid. In some embodiments, the sensor is a continuous device, for example a subcutaneous, transdermal, or intravascular device. In some embodiments, the device can analyze a plurality of intermittent blood samples. The sensor embodiments disclosed herein can use any known method, including invasive, minimally invasive, and non-invasive sensing techniques, to provide an output signal indicative of the concentration of the analyte of interest. Typically, the sensor is of the type that senses a product or reactant of an enzymatic reaction between an analyte and an enzyme in the presence of oxygen as a measure of the analyte in vivo or in vitro. Such sensors typically comprise a membrane surrounding the enzyme through which an analyte migrates. The product is then measured using electrochemical methods and thus the output of an electrode system functions as a measure of the analyte. In some aspects, the sensor can use an amperometric, coulometric, conductimetric, and/or potentiometric technique for measuring the analyte.

Embodiments of the invention disclosed herein provide sensors of the type used, for example, in subcutaneous or transcutaneous monitoring of blood glucose levels in a diabetic patient. A variety of implantable, electrochemical biosensors have been developed for the treatment of diabetes and other life-threatening diseases. Many existing sensor designs use some form of immobilized enzyme to achieve their bio-specificity. Embodiments of the invention described herein can be adapted and implemented with a wide variety of known electrochemical sensors, including for example, U.S. Patent Application No. 20050115832, U.S. Pat. Nos. 6,001,067, 6,702,857, 6,212,416, 6,119,028, 6,400,974, 6,595,919, 6,141,573, 6,122,536, 6,512,939 5,605,152, 4,431,004, 4,703,756, 6,514,718, 5,985,129, 5,390,691, 5,391, 250, 5,482,473, 5,299,571, 5,568,806, 5,494,562, 6,120,676, 6,542,765 as well as PCT International Publication Numbers WO 01/58348, WO 04/021877, WO 03/034902, WO 03/035117, WO 03/035891, WO 03/023388, WO 03/022128, WO 03/022352, WO 03/023708, WO 03/036255, WO03/036310 WO 08/042625, and WO 03/074107, and European Patent Application EP 1153571.

As discussed in detail below, embodiments of the invention disclosed herein provide sensor elements having enhanced material properties and/or architectural configurations and sensor systems (e.g. those comprising a sensor and associated electronic components such as a monitor, a processor and the like) constructed to include such elements. The disclosure further provides methods for making and using such sensors and/or architectural configurations. While some embodiments of the invention pertain to glucose and/or lactate sensors, a variety of the elements disclosed herein (e.g. interference rejection membranes) can be adapted for use with any one of the wide variety of sensors known in the art. The analyte sensor elements, architectures and methods for making and using these elements that are disclosed herein can be used to establish a variety of layered sensor structures. Such sensors of the invention exhibit a surprising degree of flexibility and versatility, characteristics which allow a wide variety of sensor configurations to be designed to examine a wide variety of analyte species.

Specific aspects of embodiments of the invention are discussed in detail in the following sections.

### I. TYPICAL ELEMENTS, CONFIGURATIONS AND ANALYTE SENSOR EMBODIMENTS OF THE INVENTION

A wide variety of sensors and sensor elements are known in the art including amperometric sensors used to detect and/or measure biological analytes such as glucose. Many glucose sensors are based on an oxygen (Clark-type) amperometric transducer (see, e.g. Yang et al., Electroanalysis 1997, 9, No. 16: 1252-1256; Clark et al., Ann. N.Y. Acad. Sci. 1962, 102, 29; Updike et al., Nature 1967, 214,986; and Wilkins et al., Med. Engin. Physics, 1996, 18, 273.3-51). A number of *in vivo* glucose sensors utilize hydrogen peroxide-based amperometric transducers because such transducers are relatively easy to fabricate and can readily be miniaturized using conventional technology. A problem associated with the use of hydrogen peroxide-based amperometric transducers, however, is signal interference due to electroactive substances present in the analyte environment. As discussed in detail below, these problems are overcome using the novel semipermeable membrane(s) disclosed herein to modulate the transport properties of different compounds that can create a signal in the hydrogen peroxide-based amperometric transducing element (e.g. electrodes of various sizes, architectures and compositions). Consequently, these membranes can be used with any of a variety of H₂O₂ based transducers used in analyte sensors that are susceptible to interference from electroactive substances.

Certain amperometric sensors comprise a plurality of layered elements including for example a base layer having an electrode, an enzyme layer and an analyte diffusion control (e.g. glucose limiting) membrane. In some sensor embodiments, an adhesion promoter layer is added to facilitate close attachment of various layers such as a diffusion control membrane and enzyme layer. One such sensor is shown in FIG. 2A. Sensors of the invention include an interference rejection membrane that is designed to inhibit and/or prevent endogenous or exogenous electro-active substances in vivo (e.g. in interstitial fluid) such as acetaminophen, uric acid and ascorbic acid from accessing the sensor electrode and being oxidized at the electrode surface (and consequently produce a spurious signal that can confound measurements of the signal generated by the analyte to be measured). One embodiment of a sensor having an interference rejection membrane is shown in FIG. 2B.

Interference rejection membranes known in the art include for example those made from materials such as cellulose acetate, NAFION (a sulfonated tetrafluoroethylene based fluoropolymer-copolymer) and electropolymerized phenylene diamine. However, these membranes exhibit a number of material properties that make them unsuitable for use with certain types of sensors. For example, while cellulose acetate compositions can function as an interference rejection membrane if appropriately formulated, the interference rejection membrane needs to be thick, usually at least 5 micrometer to get a good rejection profile. Unfortunately, this thickness requirement can add undesirable bulk to implantable sensors and can also compromise sensor operability by inhibiting an analyte's ability to generate a signal. While NAFION membranes typically do not exhibit this problem, this material is not efficient at blocking interfering molecules including acetaminophen, a major interfering species in amperometric glucose sensors. In addition, electropolymerized films such as electropolymerized phenylene diamine tend to degrade relatively quickly, thereby compromising sensor lifetimes. U.S. Patent No. 5,837,454 discloses a semi permeable membrane made via a silane condensation reaction caused by hydrolysis of alkoxy groups to yield organosilicon hydroxides which condense to form poly(organosiloxanes). Unfortunately, interference rejection membranes formed this way do not function well when disposed on coarse surfaces such as the platinum black compositions that comprise certain electrode surfaces. In addition, interference rejection membranes formed this way are relatively hydrophobic, a property which contributes to undesirable phenomena in situations where in vivo analytes are being measured in an aqueous environment (e.g. the interstitial space), for example phenomena including slow sensor hydration profiles (i.e. slow sensor initialization/wet-up) as well as sensor signal drift.

As noted above, there are a number of problems with interference rejection membranes described in the art that make them unsuitable for use with a number of sensors described in the art (e.g. amperometric glucose sensors comprising a platinum back electrode surfaces upon which a plurality of functional coatings are disposed). Consequently, there is a need in the art for interference rejection membranes having a constellation of versatile material properties that allow their use in a variety of contexts. The interference rejection membranes disclosed herein comprise polymeric materials constructed to exhibit a constellation of material properties that can be used in a variety of contexts and for example, overcome a number of technical problems observed in sensors such as electrochemical glucose sensors that are implanted in vivo and which utilize the chemical reaction between glucose and glucose oxidase to generate a measurable signal.

For example, in sensors that are implanted in vivo, an ideal interference rejection membrane should function without requiring a thick material layer that substantially increases the bulk of the sensor and/or compromises sensor operability (e.g. by effecting stiochiometric ratios of diffusing physiological reactants). In addition, for sensors such as amperometric glucose sensors, the molecular structure of the interference rejection membrane material should function as a sort of molecular sieve that inhibits the diffusion of compounds having a molecular weight greater than 140 Daltons (e.g. so as to inhibit the diffusion of compounds such as acetaminophen, uric acid, ascorbic acid and the like, while simultaneously permitting the diffusion of smaller compounds such as hydrogen peroxide). In addition to the above-noted material properties, the molecular structure of the interference rejection membrane material should be free of electrochemically reactive moieties that can for example, generate spurious signals at the surface of an electrode (either directly or indirectly). Moreover, for sensors that comprise a plurality of layered elements, the material of the interference rejection layer should exhibit adhesive properties that allow it to adhere to coarse surfaces such as the platinum black compositions that comprise some electrode surfaces while at the same time allow it to adhere to a variety of other matrices, for example adjacent layers comprising bioactive molecules such as glucose oxidase (i.e. so as to exhibit adhesive properties that inhibit delamination of the sensor layers).

In addition, sensors comprising a plurality of layers that are designed to measure analytes in aqueous environments (e.g. those implanted in vivo) typically require wetting of the layers prior to and during the measurement of accurate analyte reading. Because the properties of a material can influence the rate at which it hydrates, the material properties of a interference rejection membrane used in aqueous environments ideally will facilitate sensor wetting to, for example, minimize the time period between the sensor's introduction into an aqueous environment and its ability to provide accurate signals that correspond to the concentrations of an analyte in that environment. Embodiments of the invention that comprise the disclosed polymers crosslinked by hydrophilic compounds meet this need by facilitating sensor hydration.

Moreover, with electrochemical glucose sensors that utilize the chemical reaction between glucose and glucose oxidase to generate a measurable signal, the material of interference rejection membrane should not exacerbate (and ideally should diminish) what is known in the art as the "oxygen deficit problem". Specifically, because glucose oxidase based sensors require both oxygen (O₂) as well as glucose to generate a signal, the presence of an excess of oxygen relative to glucose, is necessary for the operation of a glucose oxidase based glucose sensor. However, because the concentration of oxygen in subcutaneous tissue is much less than that of glucose, oxygen can be the limiting reactant in the reaction between glucose, oxygen, and glucose oxidase in a sensor, a situation which compromises the sensor's ability to produce a signal that is strictly dependent on the concentration of glucose. In this context, because the properties of a material can influence the rate at which compounds diffuse through that material to the site of a measurable chemical reaction, the material properties of an interference rejection membrane used in electrochemical glucose sensors that utilize the chemical reaction between glucose and glucose oxidase to generate a measurable signal, should not for example, favor the diffusion of glucose over oxygen in a manner that contributes to the oxygen deficit problem. Sensors of the invention that comprise methacrylate or primary amine polymers crosslinked by hydrophilic compounds do not contribute to the oxygen deficit problem.

In certain embodiments of the invention, the specific placement of the interference rejection membrane element relative to other sensor elements is used to effect its function. For example, in sensor embodiments where the disclosed crosslinked polymeric compositions are disposed directly on the electroactive surface of the electrode, they function to provide size-exclusion based interference rejection membranes, ones which inhibit the diffusion of interfering species while simultaneously allowing H₂O₂ produced from an analyte enzyme reaction to access the electrode and generate an appropriate signal. Moreover, as noted above, polymers such as Poly(HEMA) and polylysine crosslinked with hydrophilic agents further provide an optimized hydrophilic environment so as to expedite the sensor initial hydration speed. Such polymers also offer a compatible matrix for a subsequent layer such as an enzyme layer (e.g. one comprising glucose oxidase) to be adhered to. Consequently, the crosslinked polymeric compositions disclosed herein exhibit a surprising constellation of material properties that make them ideal for use with certain sensor designs (e.g. implantable amperometric glucose sensors comprising a platinum back electrode surfaces upon which a plurality of functional coatings are disposed). This unexpected constellation of material properties includes for example an ability to facilitate sensor hydration (thereby decreasing start-up time), an ability to facilitate adhesion of the sensor layers so as to provide a stabilized sensor (and output signal) as well as an ability to inhibit spurious sensor signals caused by interfering species during sensor operation. The invention disclosed herein has a number of embodiments. Aspects of the disclosure include membrane compositions that can be used for example in amperometric sensors (e.g. glucose sensors used by individuals suffering from diabetes) to inhibit spurious signals caused by interfering compounds such as acetaminophen, uric acid and ascorbic acid. These interference rejection membranes typically comprise polymers crosslinked together by hydrophilic crosslinking reagent so as to facilitate sensor hydration.

One aspect of the disclosure is a polymeric composition comprising methacrylate polymers having a molecular weight between 100 and 1000 kilodaltons, wherein the methacrylate polymers are crosslinked by a hydrophilic crosslinking agent such as an organofunctional dipodal alkoxysilane. Another aspect of the disclosure is a polymeric composition comprising primary amine polymers having a molecular weight between 4,000 Daltons and 500 kilodaltons, wherein the primary amine polymers are crosslinked by a hydrophilic crosslinking agent such as glutaraldehyde. Typically these crosslinked polymeric compositions coat a hydrogen peroxide transducing composition. In an illustrative aspect, the hydrogen peroxide transducing composition comprises an electrode; and the crosslinked polymeric composition is coated on the electrode in a layer between 0.1 µm and 1.0 µm thick. A related aspect of the disclosure is a composition comprising an electrode (e.g. a platinum electrode used in an amperometric sensor) having an electroactive surface coated with, and in direct contact with a layer of crosslinked methacrylate polymers or crosslinked primary amine polymers. In certain embodiments of the invention, the IRM is designed to function (i.e. inhibit the diffusion of an interferent) where the molecular weight of the interferent is at least 140 Daltons. Typically, the IRM inhibits the diffusion of acetaminophen, ascorbic acid and/or uric acid there through to the electroactive surface of an electrode within an analyte sensor.

In certain embodiments of the invention, the crosslinked methacrylate polymers comprise Poly(2-hydroxyethyl methacrylate) polymers. As determined herein (see, e.g. Examples 1 and 2), the average molecular weight of 2-hydroxyethyl methacrylate polymers that function as an interference rejection membrane is between 100 and 1000 kilodaltons. In other embodiments of the invention, the crosslinked polymers comprise primary amine polymers (i.e. polymers comprising repeating units of primary amines such as polylysine polymers). As determined herein (see, e.g. Example 3), the average molecular weight of primary amine polymers that function as an interference rejection membrane is between 4 and 500 kilodaltons. In certain embodiments of the invention, such crosslinked polymers are adhered to a surface of an electrode that comprises an irregular architecture characteristic of an electrodeposition process (e.g. platinum black). In some embodiments, a first side of the interference rejection membrane is in direct contact with an electrochemically reactive surface of a working electrode; and an analyte sensing layer (e.g. one comprising glucose oxidase) is in direct contact with a second side of the interference rejection membrane.

Typically, the layer of polymers is disposed on a working electrode of an amperometric sensor and is crosslinked by a hydrophilic crosslinking agent that facilitates the hydration of the layer. A number of different hydrophilic crosslinking compounds useful to couple either methacrylate polymers or polyamine polymers are known in the art. Illustrative crosslinking compounds include for example, glutaraldehyde, urea; an ethylene glycol dimethacrylate; a polyethylene glycol diacrylate; an organofunctional dipodal alkoxysilane or the like. Typically, methacrylate (e.g. 2-hydroxyethyl methacrylate) polymers are crosslinked with a hydrophilic crosslinking agent that reacts with hydroxyl moieties such as an alkoxysilane crosslinker. Typically, primary amine (e.g. polylysine) polymers are crosslinked with a hydrophilic crosslinking agent that reacts with primary amine moieties such as glutaraldehyde. Suitable primary amine polymers include polylysine, poly(allylamine), Polyethylene oxide), diamine terminated, Poly(vinylamine), branched Polyethylenimine and Jeffamine Series primary amine-based oligomer or polymers etc. (and their salts). One typical compound used to make polyamine IRMs is polylysine hydrobromide having a MW of 50kd to 500kd.

One illustrative embodiment of the invention is an amperometric analyte sensor apparatus (e.g. one designed for implantation within a mammal) comprising: a base layer; a conductive layer disposed on the base layer and comprising a working electrode; an interference rejection membrane disposed on an electroactive surface of the working electrode, wherein the interference rejection membrane comprises polymers crosslinked by a hydrophilic crosslinking agent; and an analyte sensing layer (e.g. one in direct contact with the interference rejection membrane). The interference rejection membrane in the invention comprises crosslinked primary amine or crosslinked methacrylate (e.g. Poly(2-hydroxyethyl methacrylate) polymers. The crosslinked methacrylate polymers in this membrane are typically crosslinked by a hydrophilic crosslinking agent (e.g. a urea; an ethylene glycol dimethacrylate; a polyethylene glycol diacrylate; an organofunctional alkoxysilane or the like) so that the hydrophilicity of the interference rejection membrane is increased. Desirable methacrylate crosslinking agents include compounds such as organo-functional alkoxysilanes that react with organic polymers to attach the trialkoxysilyl group onto the polymer backbone. In such reactions, the silane is then available to react with moisture to crosslink the silane into a stable three-dimensional silane structure. Such mechanisms can be used to crosslink plastics, especially polyethylene, and other organic resins, such as acrylics and urethanes to impart the durability, heat resistance to coatings. In addition, crosslinkers such as a hydrophilic dipodal silane gives a double strength of crosslinking capacity while concurrently offering excellent hydrophilicity.

The crosslinked polymer compositions disclosed herein allow the design of extremely thin interference rejection membranes that do not substantially increase the thickness of an existing sensor structure. Typically the interference rejection membranes are between 0.1 to 1.0 µm thick (e.g. between 0.1 and 0.2, 0.3, 0.4, 0.5, 0.6, 0.7, 0.8, 0.9 or 1.0 µm thick), a thickness that allows them to be readily adapted for use with a variety of existing sensor designs without making substantial changes to these designs to accommodate this additional element. In preferred embodiments, the interference rejection membranes are between 0.1-0.2 µm thick. These thin interference rejection membranes can be used for example in implantable sensor embodiments of the invention to facilitate hydration of a sensor, as well as to inhibit the diffusion rate of compounds such as acetaminophen, ascorbic acid and uric acid therethrough while not substantially increasing the bulk of the implanted device (thereby decreasing the likelihood of a patient experiencing complications associated with implantation of the device). Optionally the interference rejection membranes inhibit the diffusion of acetaminophen therethrough in a manner that decreases a signal in the analyte sensor apparatus that results from a concentration of acetaminophen by at least 50% as compared to a control analyte sensor apparatus lacking the interference rejection membrane.

Embodiments of the invention include a wide variety of sensor elements and configurations of elements. For example, in certain embodiments of the invention, the interference rejection membrane is in direct contact with an electrochemically reactive surface of the working electrode; and the analyte sensing layer is disposed on the interference rejection membrane. In some embodiments of the invention, the interference rejection membrane comprises a plurality of coatings of the polymeric material (e.g. coatings disposed on an electrode via a spray process as disclosed in the Examples below). Typically the analyte sensing layer comprises an oxidoreductase (e.g. glucose oxidase) that generates hydrogen peroxide upon exposure to a substrate for the oxidoreductase (e.g. glucose), wherein the amount of hydrogen peroxide generated by the oxidoreductase is proportional to the amount of substrate exposed to the oxidoreductase. Optionally such embodiments of the invention further include: a protein layer disposed on the analyte sensing layer; an analyte modulating layer disposed on the analyte sensing layer or the protein layer, wherein the analyte modulating layer comprises a composition that modulates the diffusion of an analyte such as glucose diffusing through the analyte modulating layer; an adhesion promoting layer disposed on the analyte sensing layer, wherein the adhesion promoting layer promotes the adhesion between the analyte sensing layer and an analyte modulating layer; or a cover layer disposed on the analyte sensor apparatus, wherein the cover layer comprises an aperture positioned on the cover layer so as to facilitate an analyte present in the mammal accessing and diffusing through an analyte modulating layer; and accessing the analyte sensing layer. In some embodiments of the invention, the conductive layer comprises a plurality of electrodes including the working electrode, a counter electrode and a reference electrode. Optionally the conductive layer comprises a plurality of working electrodes, counter electrodes and reference electrodes; and the plurality of working, counter and reference electrodes are grouped together as a unit and positionally distributed on the conductive layer in a repeating pattern of units. In some embodiments of the invention, the sensor is operatively coupled to: a sensor input capable of receiving a signal from the sensor that is based on a sensed physiological characteristic value in the mammal; and a processor coupled to the sensor input, wherein the processor is capable of characterizing one or more signals received from the sensor. In certain embodiments of the invention, a pulsed voltage is used to obtain a signal from an electrode.

Another aspect of the disclosure is a method of making a sensor apparatus for implantation within a mammal comprising the steps of: providing a base layer; forming a conductive layer on the base layer, wherein the conductive layer includes a working electrode; forming an interference rejection membrane on the working electrode, wherein the interference rejection membrane comprises crosslinked methacrylate polymers or crosslinked primary amine polymers; forming an analyte sensing layer on the conductive layer, wherein the analyte sensing layer includes an oxidoreductase; optionally forming a protein layer on the analyte sensing layer; forming an adhesion promoting layer on the analyte sensing layer or the optional protein layer; forming an analyte modulating layer disposed on the adhesion promoting layer, wherein the analyte modulating layer includes a composition that modulates the diffusion of the analyte therethrough; and forming a cover layer disposed on at least a portion of the analyte modulating layer, wherein the cover layer further includes an aperture over at least a portion of the analyte modulating layer. Yet another aspect of the disclosure is a method of sensing analyte using sensors having the disclosed constellation of elements and/or made by the disclosed methodological steps.

The polymeric compositions used in the invention can be formed using a variety of art accepted processes. Typically, the interference rejection membranes are fabricated by spray method, for example as a single or multilayered coating over a substrate wafer. For example, in one illustrative embodiment of the invention, crosslinked methacrylate polymers can be formed on the electrode by a spray process using a solution comprising: 0.3 to 1% pHEMA; and 0.25% to 0.7% of a silane crosslinking agent. Alternatively, crosslinked methacrylate polymers can be formed on the electrode by a spin process using a solution comprising: pHEMA in a range from 0.25% to 4% by weight, and crosslinker content from 0.2% to 0.4% (e.g. 1 to 3% pHEMA; and 0.3% to 0.7% of a silane crosslinking agent). In yet another embodiment of the invention, crosslinked methacrylate polymers can be formed on the electrode by a slot coating process using a solution comprising: 4% pHEMA; and 0.35% to 0.7% of a silane crosslinking agent. Typically, the polymers are crosslinked, for example by exposing the polymers to a suitable crosslinking agent and then subjecting the crosslinking agent to a curing process such as heating them as described in the examples below (e.g. heating them at a range from 150°C to 220°C for 6 minutes. Primary amine based polymer compositions can be applied to surfaces such as electrodes via a spray process. A crosslinking agent such as glutaraldehyde can then be added to such polymeric materials within a chemical vapor deposition (CVD) crosslinking chamber in order to crosslink these polymers.

### A. TYPICAL SENSOR ARCHITECTURES FOUND IN EMBODIMENTS OF THE INVENTION

FIG. 2A illustrates a cross-section of a typical sensor aspect 100 of the present disclosure. This sensor is formed from a plurality of components that are typically in the form of layers of various conductive and non-conductive constituents disposed on each other according to art accepted methods and/or the specific methods disclosed herein. The components of the sensor are typically characterized herein as layers because, for example, it allows for a facile characterization of the sensor structure shown in FIG. 2. Artisans will understand however, that in certain embodiments of the invention, the sensor constituents are combined such that multiple constituents form one or more heterogeneous layers. In this context, those of skill in the art understand that the ordering of the layered constituents can be altered in various embodiments of the invention.

The aspect shown in Figure 2A includes a base layer 102 to support the sensor 100. The base layer 102 can be made of a material such as a metal and/or a ceramic and/or a polymeric substrate, which may be self-supporting or further supported by another material as is known in the art. Aspects of the disclosure include a conductive layer 104 which is disposed on and/or combined with the base layer 102. Typically the conductive layer 104 comprises one or more electrodes. An operating sensor 100 typically includes a plurality of electrodes such as a working electrode, a counter electrode and a reference electrode. Other embodiments may also include a plurality of working and/or counter and/or reference electrodes and/or one or more electrodes that performs multiple functions, for example one that functions as both as a reference and a counter electrode.

As discussed in detail below, the base layer 102 and/or conductive layer 104 can be generated using many known techniques and materials. In certain embodiments of the invention, the electrical circuit of the sensor is defined by etching the disposed conductive layer 104 into a desired pattern of conductive paths. A typical electrical circuit for the sensor 100 comprises two or more adjacent conductive paths with regions at a proximal end to form contact pads and regions at a distal end to form sensor electrodes. An electrically insulating cover layer 106 such as a polymer coating can be disposed on portions of the sensor 100. Acceptable polymer coatings for use as the insulating protective cover layer 106 can include, but are not limited to, non-toxic biocompatible polymers such as silicone compounds, polyimides, biocompatible solder masks, epoxy acrylate copolymers, or the like. In the sensors of the present invention, one or more exposed regions or apertures 108 can be made through the cover layer 106 to open the conductive layer 104 to the external environment and to, for example, allow an analyte such as glucose to permeate the layers of the sensor and be sensed by the sensing elements. Apertures 108 can be formed by a number of techniques, including laser ablation, tape masking, chemical milling or etching or photolithographic development or the like. In certain embodiments of the invention, during manufacture, a secondary photoresist can also be applied to the protective layer 106 to define the regions of the protective layer to be removed to form the aperture(s) 108. The exposed electrodes and/or contact pads can also undergo secondary processing (e.g. through the apertures 108), such as additional plating processing, to prepare the surfaces and/or strengthen the conductive regions.

In the sensor configuration shown in Figure 2A, an analyte sensing layer 110 (which is typically a sensor chemistry layer, meaning that materials in this layer undergo a chemical reaction to produce a signal that can be sensed by the conductive layer) is disposed on one or more of the exposed electrodes of the conductive layer 104. In the sensor configuration of the invention shown in Figure 2B, an interference rejection membrane 120 is disposed on one or more of the exposed electrodes of the conductive layer 104, with the analyte sensing layer 110 then being disposed on this interference rejection membrane 120. Typically, the analyte sensing layer 110 is an enzyme layer. Most typically, the analyte sensing layer 110 comprises an enzyme capable of producing and/or utilizing oxygen and/or hydrogen peroxide, for example the enzyme glucose oxidase. Optionally the enzyme in the analyte sensing layer is combined with a second carrier protein such as human serum albumin, bovine serum albumin or the like. In an illustrative embodiment, an oxidoreductase enzyme such as glucose oxidase in the analyte sensing layer 110 reacts with glucose to produce hydrogen peroxide, a compound which then modulates a current at an electrode. As this modulation of current depends on the concentration of hydrogen peroxide, and the concentration of hydrogen peroxide correlates to the concentration of glucose, the concentration of glucose can be determined by monitoring this modulation in the current. In a specific embodiment of the invention, the hydrogen peroxide is oxidized at a working electrode which is an anode (also termed herein the anodic working electrode), with the resulting current being proportional to the hydrogen peroxide concentration. Such modulations in the current caused by changing hydrogen peroxide concentrations can by monitored by any one of a variety of sensor detector apparatuses such as a universal sensor amperometric biosensor detector or one of the other variety of similar devices known in the art such as glucose monitoring devices produced by Medtronic MiniMed.

In embodiments of the invention, the analyte sensing layer 110 can be applied over portions of the conductive layer or over the entire region of the conductive layer. Typically the analyte sensing layer 110 is disposed on the working electrode which can be the anode or the cathode. Optionally, the analyte sensing layer 110 is also disposed on a counter and/or reference electrode. While the analyte sensing layer 110 can be up to about 1000 microns (µm) in thickness, typically the analyte sensing layer is relatively thin as compared to those found in sensors previously described in the art, and is for example, typically less than 1, 0.5, 0.25 or 0.1 microns in thickness. As discussed in detail below, some methods for generating a thin analyte sensing layer 110 include brushing the layer onto a substrate (e.g. the reactive surface of a platinum black electrode), as well as spin coating processes, dip and dry processes, low shear spraying processes, ink-jet printing processes, silk screen processes and the like.

Typically, the analyte sensing layer 110 is coated and or disposed next to one or more additional layers. Optionally, the one or more additional layers includes a protein layer 116 disposed upon the analyte sensing layer 110. Typically, the protein layer 116 comprises a protein such as human serum albumin, bovine serum albumin or the like. Typically, the protein layer 116 comprises human serum albumin. In some embodiments of the invention, an additional layer includes an analyte modulating layer 112 that is disposed above the analyte sensing layer 110 to regulate analyte access with the analyte sensing layer 110. For example, the analyte modulating membrane layer 112 can comprise a glucose limiting membrane, which regulates the amount of glucose that contacts an enzyme such as glucose oxidase that is present in the analyte sensing layer. Such glucose limiting membranes can be made from a wide variety of materials known to be suitable for such purposes, e.g., silicone compounds such as polydimethyl siloxanes, polyurethanes, polyurea cellulose acetates, NAFION, polyester sulfonic acids (e.g. Kodak AQ), hydrogels or any other suitable hydrophilic membranes known to those skilled in the art.

In typical embodiments of the invention, an adhesion promoter layer 114 is disposed between the analyte modulating layer 112 and the analyte sensing layer 110 as shown in FIG. 2 in order to facilitate their contact and/or adhesion. In a specific embodiment of the invention, an adhesion promoter layer 114 is disposed between the analyte modulating layer 112 and the protein layer 116 as shown in FIG. 2 in order to facilitate their contact and/or adhesion. The adhesion promoter layer 114 can be made from any one of a wide variety of materials known in the art to facilitate the bonding between such layers. Typically, the adhesion promoter layer 114 comprises a silane compound. In alternative embodiments, protein or like molecules in the analyte sensing layer 110 can be sufficiently crosslinked or otherwise prepared to allow the analyte modulating membrane layer 112 to be disposed in direct contact with the analyte sensing layer 110 in the absence of an adhesion promoter layer 114.

Embodiments of typical elements used to make the sensors disclosed herein are discussed below.

### B. TYPICAL ANALYTE SENSOR CONSTITUENTS USED IN EMBODIMENTS OF THE INVENTION

The following disclosure provides examples of typical elements/constituents used in sensor embodiments of the invention. While these elements can be described as discreet units (e.g. layers), those of skill in the art understand that sensors can be designed to contain elements having a combination of some or all of the material properties and/or functions of the elements/constituents discussed below (e.g. an element that serves both as a supporting base constituent and/or a conductive constituent and/or a matrix for the analyte sensing constituent and which further functions as an electrode in the sensor). Those in the art understand that these thin film analyte sensors can be adapted for use in a number of sensor systems such as those described below.

### BASE CONSTITUENT

Sensors of the invention include a base constituent (see, e.g. element 102 in Figure 2A). The term "base constituent" is used herein according to art accepted terminology and refers to the constituent in the apparatus that typically provides a supporting matrix for the plurality of constituents that are stacked on top of one another and comprise the functioning sensor. In one form, the base constituent comprises a thin film sheet of insulative (e.g. electrically insulative and/or water impermeable) material. This base constituent can be made of a wide variety of materials having desirable qualities such as dielectric properties, water impermeability and hermeticity. Some materials include metallic, and/or ceramic and/or polymeric substrates or the like.

The base constituent may be self-supporting or further supported by another material as is known in the art. In one aspect of the sensor configuration shown in Figure 2A, the base constituent 102 comprises a ceramic. Alternatively, the base constituent comprises a polymeric material such as a polyimmide. In an illustrative embodiment, the ceramic base comprises a composition that is predominantly Al₂O₃ (e.g. 96%). The use of alumina as an insulating base constituent for use with implantable devices is disclosed in U.S. Pat. Nos. 4,940,858, 4,678,868 and 6,472,122. The base constituents of the invention can further include other elements known in the art, for example hermetical vias (see, e.g. WO 03/023388). Depending upon the specific sensor design, the base constituent can be relatively thick constituent (e.g. thicker than 50, 100, 200, 300, 400, 500 or 1000 microns). Alternatively, one can utilize a nonconductive ceramic, such as alumina, in thin constituents, e.g., less than about 30 microns.

### CONDUCTIVE CONSTITUENT

The electrochemical sensors of the invention include a conductive constituent disposed upon the base constituent that includes at least one electrode for measuring an analyte or its byproduct (e.g. oxygen and/or hydrogen peroxide) to be assayed (see, e.g. element 104 in Figure 2A). The term "conductive constituent" is used herein according to art accepted terminology and refers to electrically conductive sensor elements such as electrodes which are capable of measuring and a detectable signal and conducting this to a detection apparatus. An illustrative example of this is a conductive constituent that can measure an increase or decrease in current in response to exposure to a stimuli such as the change in the concentration of an analyte or its byproduct as compared to a reference electrode that does not experience the change in the concentration of the analyte, a coreactant (e.g. oxygen) used when the analyte interacts with a composition (e.g. the enzyme glucose oxidase) present in analyte sensing constituent 110 or a reaction product of this interaction (e.g. hydrogen peroxide). Illustrative examples of such elements include electrodes which are capable of producing variable detectable signals in the presence of variable concentrations of molecules such as hydrogen peroxide or oxygen. Typically one of these electrodes in the conductive constituent is a working electrode, which can be made from non-corroding metal or carbon. A carbon working electrode may be vitreous or graphitic and can be made from a solid or a paste. A metallic working electrode may be made from platinum group metals, including palladium or gold, or a non-corroding metallically conducting oxide, such as ruthenium dioxide. Alternatively the electrode may comprise a silver/silver chloride electrode composition. The working electrode may be a wire or a thin conducting film applied to a substrate, for example, by coating or printing. Typically, only a portion of the surface of the metallic or carbon conductor is in electrolytic contact with the analyte-containing solution. This portion is called the working surface of the electrode. The remaining surface of the electrode is typically isolated from the solution by an electrically insulating cover constituent 106. Examples of useful materials for generating this protective cover constituent 106 include polymers such as polyimides, polytetrafluoroethylene, polyhexafluoropropylene and silicones such as polysiloxanes.

In addition to the working electrode, the analyte sensors of the invention typically include a reference electrode or a combined reference and counter electrode (also termed a quasi-reference electrode or a counter/reference electrode). If the sensor does not have a counter/reference electrode then it may include a separate counter electrode, which may be made from the same or different materials as the working electrode. Typical sensors of the present invention have one or more working electrodes and one or more counter, reference, and/or counter/reference electrodes. One embodiment of the sensor of the present invention has two, three or four or more working electrodes. These working electrodes in the sensor may be integrally connected or they may be kept separate.

Typically for in vivo use, embodiments of the present invention are implanted subcutaneously in the skin of a mammal for direct contact with the body fluids of the mammal, such as blood. Alternatively the sensors can be implanted into other regions within the body of a mammal such as in the intraperotineal space. When multiple working electrodes are used, they may be implanted together or at different positions in the body. The counter, reference, and/or counter/reference electrodes may also be implanted either proximate to the working electrode(s) or at other positions within the body of the mammal. Embodiments of the invention include sensors comprising electrodes constructed from nanostructured materials. As used herein, a "nanostructured material" is an object manufactured to have at least one dimension smaller than 100 nm. Examples include, but are not limited to, single-walled nanotubes, double-walled nanotubes, multi-walled nanotubes, bundles of nanotubes, fullerenes, cocoons, nanowires, nanofibres, onions and the like.

### INTERFERENCE REJECTION CONSTITUENT

The electrochemical sensors of the invention include an interference rejection constituent disposed between the surface of the electrode and the environment to be assayed. In particular, certain sensor embodiments rely on the oxidation and/or reduction of hydrogen peroxide generated by enzymatic reactions on the surface of a working electrode at a constant potential applied. Because amperometric detection based on direct oxidation of hydrogen peroxide requires a relatively high oxidation potential, sensors employing this detection scheme may suffer interference from oxidizable species that are present in biological fluids such as ascorbic acid, uric acid and acetaminophen. In this context, the term "interference rejection constituent" is used herein according to art accepted terminology and refers to a coating or membrane in the sensor that functions to inhibit spurious signals generated by such oxidizable species which interfere with the detection of the signal generated by the analyte to be sensed. Certain interference rejection constituents function via size exclusion (e.g. by excluding interfering species of a specific size). Examples of interference rejection constituents include one or more layers or coatings of compounds such as the hydrophilic crosslinked pHEMA and polylysine polymers disclosed herein (see, e.g. the Examples below) as well as cellulose acetate (including cellulose acetate incorporating agents such as polyethylene glycol)), polyethersulfones, polytetra-fluoroethylenes, the perfluoronated ionomer NAFION, polyphenylenediamine, epoxy and the like. Illustrative discussions of such interference rejection constituents are found for example in Ward et al., Biosensors and Bioelectronics 17 (2002) 181-189 and Choi et al., Analytical Chimica Acta 461 (2002) 251-260. Other interference rejection constituents include for example those observed to limit the movement of compounds based upon a molecular weight range, for example cellulose acetate as disclosed for example in U.S. Patent No. 5,755,939.

Additional compositions having an unexpected constellation of material properties that make them ideal for use as interference rejection membranes in certain amperometric glucose sensors as well as methods for making and using them are disclosed herein, for example in Examples 1-3 below.

In the invention the interference rejection membrane comprises crosslinked primary amine polymers or crosslinked methacrylate polymers.

### ANALYTE SENSING CONSTITUENT

The electrochemical sensors of the invention include an analyte sensing constituent disposed on the electrodes of the sensor (see, e.g. element 110 in Figure 2A). The term "analyte sensing constituent" is used herein according to art accepted terminology and refers to a constituent comprising a material that is capable of recognizing or reacting with an analyte whose presence is to be detected by the analyte sensor apparatus. Typically this material in the analyte sensing constituent produces a detectable signal after interacting with the analyte to be sensed, typically via the electrodes of the conductive constituent. In this regard the analyte sensing constituent and the electrodes of the conductive constituent work in combination to produce the electrical signal that is read by an apparatus associated with the analyte sensor. Typically, the analyte sensing constituent comprises an oxidoreductase enzyme capable of reacting with and/or producing a molecule whose change in concentration can be measured by measuring the change in the current at an electrode of the conductive constituent (e.g. oxygen and/or hydrogen peroxide), for example the enzyme glucose oxidase. An enzyme capable of producing a molecule such as hydrogen peroxide can be disposed on the electrodes according to a number of processes known in the art. The analyte sensing constituent can coat all or a portion of the various electrodes of the sensor. In this context, the analyte sensing constituent may coat the electrodes to an equivalent degree. Alternatively the analyte sensing constituent may coat different electrodes to different degrees, with for example the coated surface of the working electrode being larger than the coated surface of the counter and/or reference electrode.

Typical sensor embodiments of this element of the invention utilize an enzyme (e.g. glucose oxidase) that has been combined with a second protein (e.g. albumin) in a fixed ratio (e.g. one that is typically optimized for glucose oxidase stabilizing properties) and then applied on the surface of an electrode to form a thin enzyme constituent. In a typical embodiment, the analyte sensing constituent comprises a GOx and HSA mixture. In a typical embodiment of an analyte sensing constituent having GOx, the GOx reacts with glucose present in the sensing environment (e.g. the body of a mammal) and generates hydrogen peroxide according to the reaction shown in Figure 1, wherein the hydrogen peroxide so generated is anodically detected at the working electrode in the conductive constituent.

As noted above, the enzyme and the second protein (e.g. an albumin) are typically treated to form a crosslinked matrix (e.g. by adding a cross-linking agent to the protein mixture). As is known in the art, crosslinking conditions may be manipulated to modulate factors such as the retained biological activity of the enzyme, its mechanical and/or operational stability. Illustrative crosslinking procedures are described in U.S. Patent Application Serial Number 10/335,506 and PCT publication WO 03/035891. For example, an amine cross-linking reagent, such as, but not limited to, glutaraldehyde, can be added to the protein mixture.

### PROTEIN CONSTITUENT

The electrochemical sensors of the invention optionally include a protein constituent disposed between the analyte sensing constituent and the analyte modulating constituent (see, e.g. element 116 in Figure 2A). The term "protein constituent" is used herein according to art accepted terminology and refers to constituent containing a carrier protein or the like that is selected for compatibility with the analyte sensing constituent and/or the analyte modulating constituent. In typical embodiments, the protein constituent comprises an albumin such as human serum albumin. The HSA concentration may vary between about 0.5%-30% (w/v). Typically the HSA concentration is about 1-10% w/v, and most typically is about 5% w/v. In alternative embodiments of the invention, collagen or BSA or other structural proteins used in these contexts can be used instead of or in addition to HSA. This constituent is typically crosslinked on the analyte sensing constituent according to art accepted protocols.

### ADHESION PROMOTING CONSTITUENT

The electrochemical sensors of the invention can include one or more adhesion promoting (AP) constituents (see, e.g. element 114 in Figure 2A). The term "adhesion promoting constituent" is used herein according to art accepted terminology and refers to a constituent that includes materials selected for their ability to promote adhesion between adjoining constituents in the sensor. Typically, the adhesion promoting constituent is disposed between the analyte sensing constituent and the analyte modulating constituent. Typically, the adhesion promoting constituent is disposed between the optional protein constituent and the analyte modulating constituent. The adhesion promoter constituent can be made from any one of a wide variety of materials known in the art to facilitate the bonding between such constituents and can be applied by any one of a wide variety of methods known in the art. Typically, the adhesion promoter constituent comprises a silane compound such as γ-aminopropyltrimethoxysilane.

The use of silane coupling reagents, especially those of the formula R'Si(OR)₃ in which R' is typically an aliphatic group with a terminal amine and R is a lower alkyl group, to promote adhesion is known in the art (see, e.g. U.S. Patent No. 5,212,050). For example, chemically modified electrodes in which a silane such as γ-aminopropyltriethoxysilane and glutaraldehyde were used in a step-wise process to attach and to co-crosslink bovine serum albumin (BSA) and glucose oxidase (GOx) to the electrode surface are well known in the art (see, e.g. Yao, T. Analytica Chim. Acta 1983, 148, 27-33).

In certain embodiments of the invention, the adhesion promoting constituent further comprises one or more compounds that can also be present in an adjacent constituent such as the polydimethyl siloxane (PDMS) compounds that serves to limit the diffusion of analytes such as glucose through the analyte modulating constituent. In illustrative embodiments the formulation comprises 0.5-20% PDMS, typically 5-15% PDMS, and most typically 10% PDMS. In certain embodiments of the invention, the adhesion promoting constituent is crosslinked within the layered sensor system and correspondingly includes an agent selected for its ability to crosslink a moiety present in a proximal constituent such as the analyte modulating constituent. In illustrative embodiments of the invention, the adhesion promoting constituent includes an agent selected for its ability to crosslink an amine or carboxyl moiety of a protein present in a proximal constituent such a the analyte sensing constituent and/or the protein constituent and or a siloxane moiety present in a compound disposed in a proximal layer such as the analyte modulating layer.

### ANALYTE MODULATING CONSTITUENT

The electrochemical sensors of the invention include an analyte modulating constituent disposed on the sensor (see, e.g. element 112 in Figure 2A). The term "analyte modulating constituent" is used herein according to art accepted terminology and refers to a constituent that typically forms a membrane on the sensor that operates to modulate the diffusion of one or more analytes, such as glucose, through the constituent. In certain embodiments of the invention, the analyte modulating constituent is an analyte-limiting membrane (e.g. a glucose limiting membrane) which operates to prevent or restrict the diffusion of one or more analytes, such as glucose, through the constituents. In other embodiments of the invention, the analyte-modulating constituent operates to facilitate the diffusion of one or more analytes, through the constituents. Optionally such analyte modulating constituents can be formed to prevent or restrict the diffusion of one type of molecule through the constituent (e.g. glucose), while at the same time allowing or even facilitating the diffusion of other types of molecules through the constituent (e.g. O₂).

With respect to glucose sensors, in known enzyme electrodes, glucose and oxygen from blood, as well as some interferents, such as ascorbic acid and uric acid, diffuse through a primary membrane of the sensor. As the glucose, oxygen and interferents reach the analyte sensing constituent, an enzyme, such as glucose oxidase, catalyzes the conversion of glucose to hydrogen peroxide and gluconolactone. The hydrogen peroxide may diffuse back through the analyte modulating constituent, or it may diffuse to an electrode where it can be reacted to form oxygen and a proton to produce a current that is proportional to the glucose concentration. The sensor membrane assembly serves several functions, including selectively allowing the passage of glucose therethrough. In this context, an illustrative analyte modulating constituent is a semi-permeable membrane which permits passage of water, oxygen and at least one selective analyte and which has the ability to absorb water, the membrane having a water soluble, hydrophilic polymer.

A variety of illustrative analyte modulating compositions are known in the art and are described for example in U.S. Patent Nos. 6,319,540, 5,882,494, 5,786,439 5,777,060, 5,771,868 and 5,391,250. The hydrogels described therein are particularly useful with a variety of implantable devices for which it is advantageous to provide a surrounding water constituent. In some embodiments of the invention, the analyte modulating composition includes PDMS. In certain embodiments of the invention, the analyte modulating constituent includes an agent selected for its ability to crosslink a siloxane moiety present in a proximal constituent. In closely related embodiments of the invention, the adhesion promoting constituent includes an agent selected for its ability to crosslink an amine or carboxyl moiety of a protein present in a proximal constituent.

### COVER CONSTITUENT

The electrochemical sensors of the invention include one or more cover constituents which are typically electrically insulating protective constituents (see, e.g. element 106 in Figure 2A). Typically, such cover constituents can be in the form of a coating, sheath or tube and are disposed on at least a portion of the analyte modulating constituent. Acceptable polymer coatings for use as the insulating protective cover constituent can include, but are not limited to, non-toxic biocompatible polymers such as silicone compounds, polyimides, biocompatible solder masks, epoxy acrylate copolymers, or the like. Further, these coatings can be photo-imageable to facilitate photolithographic forming of apertures through to the conductive constituent. A typical cover constituent comprises spun on silicone. As is known in the art, this constituent can be a commercially available RTV (room temperature vulcanized) silicone composition. A typical chemistry in this context is polydimethyl siloxane (acetoxy based).

### C. TYPICAL ANALYTE SENSOR SYSTEM EMBODIMENTS OF THE INVENTION

Embodiments of the sensor elements and sensors disclosed herein can be operatively coupled to a variety of other systems elements typically used with analyte sensors (e.g. structural elements such as piercing members, insertion sets and the like as well as electronic components such as processors, monitors, medication infusion pumps and the like), for example to adapt them for use in various contexts (e.g. implantation within a mammal). One aspect of the disclosure includes a method of monitoring a physiological characteristic of a user using an embodiment of the invention that includes an input element capable of receiving a signal from a sensor that is based on a sensed physiological characteristic value of the user, and a processor for analyzing the received signal. In typical aspects, the processor determines a dynamic behavior of the physiological characteristic value and provides an observable indicator based upon the dynamic behavior of the physiological characteristic value so determined. In some aspects, the physiological characteristic value is a measure of the concentration of blood glucose in the user. In other aspects, the process of analyzing the received signal and determining a dynamic behavior includes repeatedly measuring the physiological characteristic value to obtain a series of physiological characteristic values in order to, for example, incorporate comparative redundancies into a sensor apparatus in a manner designed to provide confirmatory information on sensor function, analyte concentration measurements, the presence of interferences and the like.

Aspects of the disclosure include devices which display data from measurements of a sensed physiological characteristic (e.g. blood glucose concentrations) in a manner and format tailored to allow a user of the device to easily monitor and, if necessary, modulate the physiological status of that characteristic (e.g. modulation of blood glucose concentrations via insulin administration). An illustrative aspect of the disclosure is a device comprising a sensor input capable of receiving a signal from a sensor, the signal being based on a sensed physiological characteristic value of a user; a memory for storing a plurality of measurements of the sensed physiological characteristic value of the user from the received signal from the sensor; and a display for presenting a text and/or graphical representation of the plurality of measurements of the sensed physiological characteristic value (e.g. text, a line graph or the like, a bar graph or the like, a grid pattern or the like or a combination thereof). Typically, the graphical representation displays real time measurements of the sensed physiological characteristic value. Such devices can be used in a variety of contexts, for example in combination with other medical apparatuses. In some aspects of the disclosure, the device is used in combination with at least one other medical device (e.g. a glucose sensor).

An illustrative system consists of a glucose sensor, a transmitter and pump receiver and a glucose meter. In this system, radio signals from the transmitter can be sent to the pump receiver periodically (e.g. every 5 minutes) to provide providing real-time sensor glucose (SG) values. Values/graphs are displayed on a monitor of the pump receiver so that a user can self monitor blood glucose and deliver insulin using their own insulin pump. Typically an aspect of device disclosed herein communicates with a second medical device via a wired or wireless connection. Wireless communication can include for example the reception of emitted radiation signals as occurs with the transmission of signals via RF telemetry, infrared transmissions, optical transmission, sonic and ultrasonic transmissions and the like. Optionally, the device is an integral part of a medication infusion pump (e.g. an insulin pump). Typically in such devices, the physiological characteristic values includes a plurality of measurements of blood glucose.

### D. EMBODIMENTS OF THE INVENTION AND ASSOCIATED

### CHARACTERISTICS

Embodiments of the invention disclosed herein focus on implantable analyte sensors and sensor systems that are designed to include hydrophilic compositions (e.g. an interference rejection membrane comprising polymers crosslinked by a hydrophilic crosslinking agent) and/or configurations of elements that facilitate sensor initialization and/or start-up in vivo (e.g. the run-in time that it takes for a sensor to settle into its aqueous environment and start transmitting meaningful information after being implanted in vivo). In particular, it is known in the art that the amount time required for sensor initialization and/or start-up prior to its use can be relatively long (e.g. in amperometric glucose sensors, the sensor start-up initialization times can range from 2 to 10 hours), a factor which can hinder the use of such sensors in the administration of medical care. For example, in hospital settings, a relatively long sensor initialization and/or start-up period can delay the receipt of important information relating to patient health (e.g. hyperglycemia or hypoglycemia in a diabetic patient), thereby delaying treatments predicated on the receipt of such information (e.g. the administration of insulin). In addition, a relatively long sensor initialization and/or start-up period in hospital settings can require repeated monitoring by hospital staff, a factor which contributes to the costs of patient care. For these reasons, sensors having reduced initialization and/or start-up times in vivo in hospital settings and sensors and sensor systems that are designed to include elements and/or configurations of elements that diminish long sensor initialization and/or start-up times are highly desirable. With glucose sensors for example, a 15-30 minute reduction of sensor initialization and/or start-up time is highly desirable because, for example, such shorter initialization times can: (1) reduce the need for patient monitoring by hospital personnel, a factor which contributes to the cost-effectiveness of such medical devices; and (2) reduce delays in the receipt of important information relating to patient health.

In individuals using analyte sensors in non-hospital settings (e.g. diabetics using glucose sensors to manage their disease), relatively long sensor initialization and/or start-up periods are also problematical due to both the inconvenience to the user as well as the delayed receipt of information relating to user health. The use of glucose sensors, insulin infusion pumps and the like in the management of diabetes has increased in recent years due for example to studies showing that the morbidity and mortality issues associated with this chronic disease decrease dramatically when a patient administers insulin in a manner that closely matches the rise and fall of physiological insulin concentrations in healthy individuals. Consequently, patients who suffer from chronic diseases such as diabetes are instructed by medical personnel to play an active role in the management of their disease, in particular, the close monitoring and modulation of blood glucose levels. In this context, because many diabetics do not have medical training, they may forgo optimal monitoring and modulation of blood glucose levels due to complexities associated with such management, for example, a two hour start-up period which can be an inconvenience in view of a patient's active daily routine. For these reasons, sensors and sensor systems that are designed to include elements and/or configurations of elements can reduce sensor initialization and/or start-up times (e.g. the hydrophilic interference rejection membranes disclosed herein) are highly desirable in situations where such sensors are operated by a diabetic patient without medical training because they facilitate the patient's convenient management of their disease, behavior which is shown to decrease the well known morbidity and mortality issues observed in individuals suffering from chronic diabetes.

While the analyte sensor and sensor systems disclosed herein are typically designed to be implantable within the body of a mammal, the inventions disclosed herein are not limited to any particular environment and can instead be used in a wide variety of contexts, for example for the analysis of most in vivo and in vitro liquid samples including biological fluids such as interstitial fluids, whole-blood, lymph, plasma, serum, saliva, urine, stool, perspiration, mucus, tears, cerebrospinal fluid, nasal secretion, cervical or vaginal secretion, semen, pleural fluid, amniotic fluid, peritoneal fluid, middle ear fluid, joint fluid, gastric aspirate or the like. In addition, solid or desiccated samples may be dissolved in an appropriate solvent to provide a liquid mixture suitable for analysis.

The invention disclosed herein has a number of embodiments. One illustrative embodiment of the invention is an analyte sensor apparatus comprising: an elongated (i.e. having notably more length than width) base layer; a conductive layer disposed on the base layer and comprising a reference electrode, a working electrode and a counter electrode; an interference rejection membrane disposed upon the conductive layer, an analyte sensing layer disposed on the interference rejection membrane; an analyte modulating layer disposed on the analyte sensing layer, wherein the analyte modulating layer comprises a composition that modulates the diffusion of an analyte diffusing through the analyte modulating layer; and a cover layer disposed on the analyte sensor apparatus, wherein the cover layer comprises an aperture positioned on the cover layer so as to facilitate an analyte accessing and diffusing through the analyte modulating layer and accessing the analyte sensing layer. Typical embodiments of the invention are comprised of biocompatible materials and/or have structural features designed for implantation within a mammal. Methodological aspects of the disclosure include methods for making and using the sensor embodiments disclosed herein. Certain aspects of the disclosure include methods of using a specific sensor element and/or a specific constellation of sensor elements to produce and/or facilitate one or more functions of the sensor embodiments disclosed herein.

As disclosed herein, those of skill in the art understand that a conductive layer disposed on the base layer and comprising a working electrode, a counter electrode and a reference electrode includes embodiments wherein the conductive layer is disposed on at least a portion the base layer and does not necessarily completely cover the base layer. Those of skill in the art will understand that this refers to other layers within the sensor, with for example, an analyte sensing layer disposed on the conductive layer encompassing sensor embodiments where the analyte sensing layer disposed on at least a portion of the conductive layer; and an analyte modulating layer disposed on the analyte sensing encompassing an analyte modulating layer disposed on at least a portion of the analyte sensing etc. etc. Optionally, the electrodes can be disposed on a single surface or side of the sensor structure. Alternatively, the electrodes can be disposed on a multiple surfaces or sides of the sensor structure (and can for example be connected by vias through the sensor material(s) to the surfaces on which the electrodes are disposed). In certain embodiments of the invention, the reactive surfaces of the electrodes are of different relative areas/sizes, for example a 1X reference electrode, a 2.6X working electrode and a 3.6X counter electrode.

In certain embodiments of the invention, an element of the apparatus such as an electrode or an aperture is designed to have a specific configuration and/or is made from a specific material and/or is positioned relative to the other elements so as to facilitate a function of the sensor. For example, without being bound by a specific theory or mechanism of action, it appears that sensor embodiments (e.g. simple three electrode embodiments) may be more susceptible to local environment changes around a single electrode. For example, a gas bubble on top of or close to a reference or another electrode, and/or a stagnating or semi-stagnating pool of fluid on top of or close to a reference or another electrode may consequently compromises sensor performance. In this context, a distributed electrode configuration appears be advantageous because the distribution of the electrode area allows the sensor to compensate for signal lost to a small local area (e.g. as can occur due to lack of hydration, fluid stagnation, a patient's immune response, or the like).

Typical analyte sensor apparatus embodiments comprises a plurality of working electrodes, counter electrodes and reference electrodes. Optionally, the plurality of working, counter and reference electrodes are grouped together as a unit and positionally distributed on the conductive layer in a repeating pattern of units. Alternatively, the plurality of working, counter and reference electrodes are grouped together and positionally distributed on the conductive layer in a non-repeating pattern of units. In certain embodiments of the invention, the elongated base layer is made from a material that allows the sensor to twist and bend when implanted in vivo; and the electrodes are grouped in a configuration that facilitates an in vivo fluid accessing at least one of working electrode as the sensor apparatus twists and bends when implanted in vivo. In some embodiments, the electrodes are grouped in a configuration that allows the sensor to continue to maintain an optimal function if a portion of the sensor having one or more electrodes is dislodged from an in vivo environment and exposed to an ex vivo environment.

Optionally, embodiments of the invention include a plurality of working electrodes and/or counter electrodes and/or reference electrodes (e.g. to provide redundant sensing capabilities). Such embodiments of the invention can be used in embodiments of the invention that include a processor (e.g. one linked to a program adapted for a signal subtraction/cancellation process) are designed factor out background signals in vivo, for example by comparing signal(s) at GOx coated working electrode with signal at working electrode not coated with GOx (e.g. background detection followed by a signal subtraction/cancellation process to arrive at a true signal). Certain of these embodiments of the invention are particularly useful for sensing glucose at the upper and lower ends of the glucose signal curves. Similar embodiments of the invention are used to factor out interference, for example by comparing signal(s) at GOx coated working electrode with signal at working electrode not coated with GOx. Embodiments of the invention can include a coating of a Prussian blue composition on an electrode at a location and in an amount sufficient to mediate an electrical potential of an electrode of the apparatus. Related embodiments of the invention include methods for mediating an electrical potential of an electrode of the disclosed sensor apparatus (e.g. by using a Prussian blue composition). Prussian Blue formulas are known in the art and include Fe4[Fe(CN6]3 ×H20, CI no. 77510 and KFe[Fe(Cn)6] ×H20 id CI no. 77520.

In typical embodiments of the invention, the sensor is operatively coupled to further elements (e.g. electronic components) such as elements designed to transmit and/or receive a signal, monitors, pumps, processors and the like. For example, in some embodiments of the invention, the sensor is operatively coupled to a sensor input capable of receiving a signal from the sensor that is based on a sensed physiological characteristic value in the mammal; and a processor coupled to the sensor input, wherein the processor is capable of characterizing one or more signals received from the sensor. A wide variety of sensor configurations as disclosed herein can be used in such systems. Optionally, for example, the sensor comprises three working electrodes, one counter electrode and one reference electrode. In certain embodiments, at least one working electrode is coated with an analyte sensing layer comprising glucose oxidase (and optionally two are coated with GOx) and at least one working electrode is not coated with an analyte sensing layer comprising glucose oxidase. Such embodiments of the invention can be used for example in sensor embodiments designed factor out background signals in vivo, for example by comparing signal(s) at GOx coated working electrode(s) with signal at working electrode(s) not coated with GOx (e.g. background detection followed by a signal subtraction/cancellation process to arrive at a true signal).

In some embodiments of sensors insertion set apparatuses, a first and a second (and/or third etc.) electrochemical sensor comprises one working, counter and reference electrode. Alternatively, the plurality of electrochemical sensors comprise a plurality of working, counter and reference electrodes, for example those having a distributed configuration as disclosed in U.S. Patent Application Serial No. 11/633,254. In certain embodiments of the invention, at least two in the plurality of sensors are designed to measure a signal generated by the same physiological characteristic, for example blood glucose concentration. Embodiments of the invention can include for example a plurality of electrochemical sensors having a working electrode coated with an oxidoreductase such as glucose oxidase and are used in methods designed to sample and compare glucose concentrations observed at the plurality of in vivo insertion sites. Alternatively, at least two in the plurality of sensors in the sensor apparatus are designed to measure signals generated by the different characteristics, for example a first characteristic comprising a background or interfering signal that is unrelated to blood glucose (e.g. "interferent noise") and a second characteristic comprising blood glucose concentrations. In an illustrative embodiment of this invention, a first sensor is designed to measure glucose oxidase and comprises one or more working electrodes coated with glucose oxidase while a second comparative sensor is designed to measure a background or interfering signal that is unrelated to blood glucose has no working electrode (or electrodes) coated with glucose oxidase.

In certain embodiments of the invention, sensor systems that utilize voltage pulsing and/or switching as disclosed herein are used in methods designed to overcome problems that can occur with implantable sensors and sensor systems due to lack of hydration (e.g. slow start-up initialization times) and/or fluid stagnation by enhancing the ability of a fluid to flow around the implanted components in a manner that inhibits the likelihood of a gas bubble or a stagnating pool of fluid from forming and/or remaining on top of or close to an electrode in a manner that compromises sensor function. In addition, embodiments of the invention that utilize voltage pulsing and/or switching can be combined with certain complementary elements disclosed herein so as to further overcome problems that result from a lack of hydration, fluid stagnation, a patient's immune response, or the like (e.g. distributed electrode configurations, multiple electrode sensors, multiple sensor apparatuses having multiple implantation sites, etc.).

In some embodiments of the invention, a processor is capable of comparing a first signal received from a working electrode in response to a first working potential with a second signal received from a working electrode in response to a second working potential, wherein the comparison of the first and second signals at the first and second working potentials can be used to identify a signal generated by an interfering compound. In one such embodiment of the invention, one working electrode is coated with glucose oxidase and another is not, and the interfering compound is acetaminophen, ascorbic acid, bilirubin, cholesterol, creatinine, dopamine, ephedrine, ibuprofen, L-dopa, methyldopa, salicylate, tetracycline, tolazamide, tolbutamide, triglycerides or uric acid. Optionally, a pulsed and/or varied (e.g. switched) voltage is used to obtain a signal from a working electrode. Typically, at least one voltage is 280, 535 or 635 millivolts. Related aspects of the disclosure include methods for identifying and/or characterizing one or more signals generated by an interfering compound in various sensor embodiments of the invention (e.g. by comparing the signal from an electrode coated with an analyte sensing compound with a comparative electrode not coated with an analyte sensing compound). Optionally, such methods use a pulsed and/or varied working potential to observe a signal at an electrode.

Sensors of the invention can also be incorporated in to a wide variety of medical systems known in the art. Sensors of the invention can be used, for example, in a closed loop infusion systems designed to control the rate that medication is infused into the body of a user. Such a closed loop infusion system can include a sensor and an associated meter which generates an input to a controller which in turn operates a delivery system (e.g. one that calculates a dose to be delivered by a medication infusion pump). In such contexts, the meter associated with the sensor may also transmit commands to, and be used to remotely control, the delivery system. Typically, the sensor is a subcutaneous sensor in contact with interstitial fluid to monitor the glucose concentration in the body of the user, and the liquid infused by the delivery system into the body of the user includes insulin. Illustrative systems are disclosed for example in U. S. Patent Nos. 6,558,351 and 6,551,276; PCT Application Nos. US99/21703 and US99/22993; as well as WO 2004/008956 and WO 2004/009161.

Certain embodiments of the invention measure peroxide and have the advantageous characteristic of being suited for implantation in a variety of sites in the mammal including regions of subcutaneous implantation and intravenous implantation as well as implantation into a variety of non-vascular regions. A peroxide sensor design that allows implantation into non-vascular regions has advantages over certain sensor apparatus designs that measure oxygen due to the problems with oxygen noise that can occur in oxygen sensors implanted into non-vascular regions. For example, in such implanted oxygen sensor apparatus designs, oxygen noise at the reference sensor can compromise the signal to noise ratio which consequently perturbs their ability to obtain stable glucose readings in this environment. The sensors of the invention therefore overcome the difficulties observed with such oxygen sensors in non-vascular regions.

In some embodiments of the invention, the analyte sensor apparatus is designed to function via anodic polarization such that the alteration in current is detected at the anodic working electrode in the conductive layer of the analyte sensor apparatus. Structural design features that can be associated with anodic polarization include designing an appropriate sensor configuration comprising a working electrode which is an anode, a counter electrode which is a cathode and a reference electrode, and then selectively disposing the appropriate analyte sensing layer on the appropriate portion of the surface of the anode within this design configuration. Optionally this anodic polarization structural design includes anodes, cathodes and/or working electrodes having different sized surface areas. For example, this structural design includes features where the working electrode (anode) and/or the coated surface of the working electrode is larger or smaller than the counter electrode (cathode) and/or the coated surface of the counter electrode (e.g. a sensor designed to have a 1X area for a reference electrode, a 2.6X area for a working electrode and a 3.6X area for a counter electrode). In this context, the alteration in current that can be detected at the anodic working electrode is then correlated with the concentration of the analyte. In certain illustrative examples of this embodiment of the invention, the working electrode is measuring and utilizing hydrogen peroxide in the oxidation reaction (see e.g. FIG. 1), hydrogen peroxide that is produced by an enzyme such as glucose oxidase or lactate oxidase upon reaction with glucose or lactate respectively.

### II. ILLUSTRATIVE METHODS AND MATERIALS FOR MAKING ANALYTE SENSOR APPARATUS OF THE INVENTION

A number of articles, U.S. patents and patent application describe the state of the art with the common methods and materials disclosed herein and further describe various elements (and methods for their manufacture) that can be used in the sensor designs disclosed herein. These include for example, U.S. Patent Nos. 6,413,393; 6,368,274; 5,786,439; 5,777,060; 5,391,250; 5,390,671; 5,165,407, 4,890,620, 5,390,671, 5,390,691, 5,391,250, 5,482,473, 5,299,571, 5,568,806; United States Patent Application 20020090738; as well as PCT International Publication Numbers WO 01/58348, WO 03/034902, WO 03/035117, WO 03/035891, WO 03/023388, WO 03/022128, WO 03/022352, WO 03/023708, WO 03/036255, WO03/036310 and WO 03/074107.

Typical sensors for monitoring glucose concentration of diabetics are further described in Shichiri, et al.,: "In Vivo Characteristics of Needle-Type Glucose Sensor-Measurements of Subcutaneous Glucose Concentrations in Human Volunteers," Horm. Metab. Res., Suppl. Ser. 20:17-20 (1988); Bruckel, et al.,: "In Vivo Measurement of Subcutaneous Glucose Concentrations with an Enzymatic Glucose Sensor and a Wick Method," Klin. Wochenschr. 67:491-495 (1989); and Pickup, et al.,: "In Vivo Molecular Sensing in Diabetes Mellitus: An Implantable Glucose Sensor with Direct Electron Transfer," Diabetologia 32:213-217 (1989). Other sensors are described in, for example Reach, et al., in ADVANCES IN IMPLANTABLE DEVICES, A. Turner (ed.), JAI Press, London, Chap. 1, (1993).

### A. GENERAL METHODS FOR MAKING ANALYTE SENSORS

A typical aspect disclosed herein is a method of making a sensor apparatus for implantation within a mammal comprising the steps of: providing a base layer; forming a conductive layer on the base layer, wherein the conductive layer includes an electrode (and typically a working electrode, a reference electrode and a counter electrode); forming a interference rejection membrane on the conductive layer, forming an analyte sensing layer on the interference rejection membrane, wherein the analyte sensing layer includes a composition that can alter the electrical current at the electrode in the conductive layer in the presence of an analyte; optionally forming a protein layer on the analyte sensing layer; forming an adhesion promoting layer on the analyte sensing layer or the optional protein layer; forming an analyte modulating layer disposed on the adhesion promoting layer, wherein the analyte modulating layer includes a composition that modulates the diffusion of the analyte therethrough; and forming a cover layer disposed on at least a portion of the analyte modulating layer, wherein the cover layer further includes an aperture over at least a portion of the analyte modulating layer. In certain embodiments of the invention, the analyte modulating layer comprises a hydrophilic comb-copolymer having a central chain and a plurality of side chains coupled to the central chain, wherein at least one side chain comprises a silicone moiety. In some aspects of these methods, the analyte sensor apparatus is formed in a planar geometric configuration

As disclosed herein, the various layers of the sensor can be manufactured to exhibit a variety of different characteristics which can be manipulated according to the specific design of the sensor. For example, the adhesion promoting layer includes a compound selected for its ability to stabilize the overall sensor structure, typically a silane composition. In some embodiments of the invention, the analyte sensing layer is formed by a spin coating process and is of a thickness selected from the group consisting of less than 1, 0.5, 0.25 and 0.1 microns in height.

Typically, a method of making the sensor includes the step of forming a protein layer on the analyte sensing layer, wherein a protein within the protein layer is an albumin selected from the group consisting of bovine serum albumin and human serum albumin. Typically, a method of making the sensor includes the step of forming an analyte sensing layer that comprises an enzyme composition selected from the group consisting of glucose oxidase, glucose dehydrogenase, lactate oxidase, hexokinase and lactate dehydrogenase. In such methods, the analyte sensing layer typically comprises a carrier protein composition in a substantially fixed ratio with the enzyme, and the enzyme and the carrier protein are distributed in a substantially uniform manner throughout the analyte sensing layer.

### B. TYPICAL PROTOCOLS AND MATERIALS USEFUL IN THE MANUFACTURE OF ANALYTE SENSORS

The disclosure provided herein includes sensors and sensor designs that can be generated using combinations of various well known techniques. The disclosure further provides methods for applying very thin enzyme coatings to these types of sensors as well as sensors produced by such processes. In this context, some aspects of the disclosure include methods for making such sensors on a substrate according to art accepted processes. In certain embodiments, the substrate comprises a rigid and flat structure suitable for use in photolithographic mask and etch processes. In this regard, the substrate typically defines an upper surface having a high degree of uniform flatness. A polished glass plate may be used to define the smooth upper surface. Alternative substrate materials include, for example, stainless steel, aluminum, and plastic materials such as delrin, etc. In other embodiments, the substrate is non-rigid and can be another layer of film or insulation that is used as a substrate, for example plastics such as polyimides and the like.

An initial step in the methods typically includes the formation of a base layer of the sensor. The base layer can be disposed on the substrate by any desired means, for example by controlled spin coating. In addition, an adhesive may be used if there is not sufficient adhesion between the substrate layer and the base layer. A base layer of insulative material is formed on the substrate, typically by applying the base layer material onto the substrate in liquid form and thereafter spinning the substrate to yield the base layer of thin, substantially uniform thickness. These steps are repeated to build up the base layer of sufficient thickness, followed by a sequence of photolithographic and/or chemical mask and etch steps to form the conductors discussed below. In an illustrative form, the base layer comprises a thin film sheet of insulative material, such as ceramic or polyimide substrate. The base layer can comprise an alumina substrate, a polyimide substrate, a glass sheet, controlled pore glass, or a planarized plastic liquid crystal polymer. The base layer may be derived from any material containing one or more of a variety of elements including, but not limited to, carbon, nitrogen, oxygen, silicon, sapphire, diamond, aluminum, copper, gallium, arsenic, lanthanum, neodymium, strontium, titanium, yttrium, or combinations thereof. Additionally, the substrate may be coated onto a solid support by a variety of methods well-known in the art including physical vapor deposition, or spin-coating with materials such as spin glasses, chalcogenides, graphite, silicon dioxide, organic synthetic polymers, and the like.

The methods further include the generation of a conductive layer having one or more sensing elements. Typically these sensing elements are electrodes that are formed by one of the variety of methods known in the art such as photoresist, etching and rinsing to define the geometry of the active electrodes. The electrodes can then be made electrochemically active, for example by electrodeposition of Pt black for the working and counter electrode, and silver followed by silver chloride on the reference electrode. A sensor layer such as a analyte sensing enzyme layer can then be disposed on the sensing layer by electrochemical deposition or a method other than electrochemical deposition such a spin coating, followed by vapor crosslinking, for example with a dialdehyde (glutaraldehyde) or a carbodi-imide.

Electrodes of the sensors of the invention can be formed from a wide variety of materials known in the art. For example, the electrode may be made of a noble late transition metals. Metals such as gold, platinum, silver, rhodium, iridium, ruthenium, palladium, or osmium can be suitable in various embodiments of the invention. Other compositions such as carbon or mercury can also be useful in certain sensor embodiments. Of these metals, silver, gold, or platinum is typically used as a reference electrode metal. A silver electrode which is subsequently chloridized is typically used as the reference electrode. These metals can be deposited by any means known in the art, including the plasma deposition method cited, supra, or by an electroless method which may involve the deposition of a metal onto a previously metallized region when the substrate is dipped into a solution containing a metal salt and a reducing agent. The electroless method proceeds as the reducing agent donates electrons to the conductive (metallized) surface with the concomitant reduction of the metal salt at the conductive surface. The result is a layer of adsorbed metal. (For additional discussions on electroless methods, see: Wise, E. M. Palladium: Recovery, Properties, and Uses, Academic Press, New York, New York (1988); Wong, K. et al. Plating and Surface Finishing 1988, 75, 70-76; Matsuoka, M. et al. Ibid. 1988, 75, 102-106; and Pearlstein, F. "Electroless Plating," Modern Electroplating, Lowenheim, F. A., Ed., Wiley, New York, N.Y. (1974), Chapter 31.). Such a metal deposition process must yield a structure with good metal to metal adhesion and minimal surface contamination, however, to provide a catalytic metal electrode surface with a high density of active sites. Such a high density of active sites is a property necessary for the efficient redox conversion of an electroactive species such as hydrogen peroxide.

In an exemplary embodiment of the invention, the base layer is initially coated with a thin film conductive layer by electrode deposition, surface sputtering, or other suitable process step. In one embodiment this conductive layer may be provided as a plurality of thin film conductive layers, such as an initial chrome-based layer suitable for chemical adhesion to a polyimide base layer followed by subsequent formation of thin film gold-based and chrome-based layers in sequence. In alternative embodiments, other electrode layer conformations or materials can be used. The conductive layer is then covered, in accordance with conventional photolithographic techniques, with a selected photoresist coating, and a contact mask can be applied over the photoresist coating for suitable photoimaging. The contact mask typically includes one or more conductor trace patterns for appropriate exposure of the photoresist coating, followed by an etch step resulting in a plurality of conductive sensor traces remaining on the base layer. In an illustrative sensor construction designed for use as a subcutaneous glucose sensor, each sensor trace can include three parallel sensor elements corresponding with three separate electrodes such as a working electrode, a counter electrode and a reference electrode.

Portions of the conductive sensor layers are typically covered by an insulative cover layer, typically of a material such as a silicon polymer and/or a polyimide. The insulative cover layer can be applied in any desired manner. In an exemplary procedure, the insulative cover layer is applied in a liquid layer over the sensor traces, after which the substrate is spun to distribute the liquid material as a thin film overlying the sensor traces and extending beyond the marginal edges of the sensor traces in sealed contact with the base layer. This liquid material can then be subjected to one or more suitable radiation and/or chemical and/or heat curing steps as are known in the art. In alternative embodiments, the liquid material can be applied using spray techniques or any other desired means of application. Various insulative layer materials may be used such as photoimagable epoxyacrylate, with an illustrative material comprising a photoimagable polyimide available from OCG, Inc. of West Paterson, N.J., under the product number 7020.

Sensors of the invention have an interference rejection layer on the conductive layer. This may be formed for example using a methodology disclosed in Examples 1-3 below. Briefly for example, one can form an interference rejection membrane using a pHEMA composition comprising 95% C2H5OH and 5% H2O as the solvent system for pHEMA for a spray application process. Alternatively 95% 2-Butanol and 5% H2O can be another choice for a spray application process, on that exhibits slower evaporation. In such application processes, the concentration of pHEMA typically ranges from 0.25% to 4% by weight, with crosslinker content typically ranging from 0.2% to 0.4% (which can, for example, be determined via the dispensing method). Such processes can include a heating/curing step. In typical embodiments, the heating temperature for curing the membrane can range from 150°C to 220°C for a time around 2-15 minutes (e.g. 6 minutes). A wide variety of permutations of such methods will be apparent to artisans. For example in some embodiments, on can apply the interference rejection membrane by spraying a 0.3-1% Mw 300kd pHEMA solution (e.g. 0.5%) and 0.25% to 0.4% (0.275%) silane crosslinker. Alternatively, artisans can use a spin coating process with a 1-3% 300kd pHEMA solution and a silane crosslinker concentration of 0.3% to 0.4%, at a spin speed of1000rpm for 30 seconds to generate a single layer. Alternatively, artisans can use a slot coating process with a 4% 200kd pHEMA solution and a silane crosslinker concentration of 0.35% to 0.5%, and 2 pass coating process to generate multiple layers.

In an illustrative sensor embodiment for use as a glucose sensor, an enzyme (typically glucose oxidase) is coated with the enzyme so as to define a working electrode. One or both of the other electrodes can be provided with the same coating as the working electrode. Alternatively, the other two electrodes can be provided with other suitable chemistries, such as other enzymes, left uncoated, or provided with chemistries to define a reference electrode and a counter electrode for the electrochemical sensor. Methods for producing the enzyme coatings include spin coating processes, dip and dry processes, low shear spraying processes, ink-jet printing processes, silk screen processes and the like. Typically, such coatings are vapor crosslinked subsequent to their application. Surprisingly, sensors produced by these processes have material properties that exceed those of sensors having coatings produced by electrodeposition including enhanced longevity, linearity, regularity as well as improved signal to noise ratios. In addition, embodiments of the invention that utilize glucose oxidase coatings formed by such processes are designed to recycle hydrogen peroxide and improve the biocompatibility profiles of such sensors.

Sensors generated by processes such as spin coating processes also avoid other problems associated with electrodeposition, such as those pertaining to the material stresses placed on the sensor during the electrodeposition process. In particular, the process of electrodeposition is observed to produce mechanical stresses on the sensor, for example mechanical stresses that result from tensile and/or compression forces. In certain contexts, such mechanical stresses may result in sensors having coatings with some tendency to crack or delaminate. This is not observed in coatings disposed on sensor via spin coating or other low-stress processes.

In the invention, the sensor may be made by methods which apply an analyte modulating layer that comprises a hydrophilic membrane coating which can regulate the amount of analyte that can access the enzyme of the sensor layer. For example, the cover layer that is added to the glucose sensors of the invention can comprise a glucose limiting membrane, which regulates the amount of glucose that access glucose oxidase enzyme layer on an electrode. Such glucose limiting membranes can be made from a wide variety of materials known to be suitable for such purposes, e.g., silicones such as polydimethyl siloxane and the like, polyurethanes, cellulose acetates, NAFION, polyester sulfonic acids (e.g. Kodak AQ), hydrogels or any other membrane known to those skilled in the art that is suitable for such purposes. In certain embodiments of the invention, the analyte modulating layer comprises a hydrophilic comb-copolymer having a central chain and a plurality of side chains coupled to the central chain, wherein at least one side chain comprises a silicone moiety. In some embodiments of the invention pertaining to sensors having hydrogen peroxide recycling capabilities, the membrane layer that is disposed on the glucose oxidase enzyme layer functions to inhibit the release of hydrogen peroxide into the environment in which the sensor is placed and to facilitate hydrogen peroxide molecules and accessing the electrode sensing elements.

In some embodiments, an adhesion promoter layer is disposed between a cover layer (e.g. an analyte modulating membrane layer) and a analyte sensing layer in order to facilitate their contact and is selected for its ability to increase the stability of the sensor apparatus. As noted herein, compositions of the adhesion promoter layer are selected to provide a number of desirable characteristics in addition to an ability to provide sensor stability. For example, some compositions for use in the adhesion promoter layer are selected to play a role in interference rejection as well as to control mass transfer of the desired analyte. The adhesion promoter layer can be made from any one of a wide variety of materials known in the art to facilitate the bonding between such layers and can be applied by any one of a wide variety of methods known in the art. Typically, the adhesion promoter layer comprises a silane compound such as γ-aminopropyltrimethoxysilane. In certain embodiments of the invention, the adhesion promoting layer and/or the analyte modulating layer comprises an agent selected for its ability to crosslink a siloxane moiety present in a proximal. In other embodiments of the invention, the adhesion promoting layer and/or the analyte modulating layer comprises an agent selected for its ability to crosslink an amine or carboxyl moiety of a protein present in a proximal layer. In an optional embodiment, the AP layer further comprises Polydimethyl Siloxane (PDMS), a polymer typically present in analyte modulating layers such as a glucose limiting membrane. In illustrative embodiments the formulation comprises 0.5-20% PDMS, typically 5-15% PDMS, and most typically 10% PDMS. The addition of PDMS to the AP layer can be advantageous in contexts where it diminishes the possibility of holes or gaps occurring in the AP layer as the sensor is manufactured.

A sensor electrode of the invention may be made by providing an electroactive surface which can function as an electrode (e.g. platinum), forming an interference rejection membrane on the electroactive surface, spin coating an enzyme layer on the IRM and then forming an analyte contacting layer (e.g. an analyte modulating layer such as a glucose limiting membrane) on the electrode, wherein the analyte contacting layer regulates the amount of analyte that can contact the enzyme layer. In some methods, the enzyme layer is vapor crosslinked on the sensor layer. In a typical embodiment of the invention, a sensor is formed to include at least one working electrode and at least one counter electrode. In certain embodiments, the IRM is formed on at least a portion of the working electrode and at least a portion of the counter electrode. Typically, the enzyme layer comprises one or more enzymes such as glucose oxidase, glucose dehydrogenase, lactate oxidase, hexokinase or lactate dehydrogenase and/or like enzymes. In a specific method, the enzyme layer comprises glucose oxidase that is stabilized by coating it on the sensor layer in combination with a carrier protein in a fixed ratio. Typically the carrier protein is albumin. Typically such methods include the step of forming an adhesion promoter layer disposed between the glucose oxidase layer and the analyte contacting layer. Optionally, a layer such as the IRM and/or the adhesion promoter layer is subjected to a curing process prior to the formation of the analyte contacting layer.

The finished sensors produced by such processes are typically quickly and easily removed from a supporting substrate (if one is used), for example, by cutting along a line surrounding each sensor on the substrate. The cutting step can use methods typically used in this art such as those that include a UV laser cutting device that is used to cut through the base and cover layers and the functional coating layers along a line surrounding or circumscribing each sensor, typically in at least slight outward spaced relation from the conductive elements so that the sufficient interconnected base and cover layer material remains to seal the side edges of the finished sensor. In addition, dicing techniques typically used to cut ceramic substrates can be used with the appropriate sensor embodiments. Since the base layer is typically not physically attached or only minimally adhered directly to the underlying supporting substrate, the sensors can be lifted quickly and easily from the supporting substrate, without significant further processing steps or potential damage due to stresses incurred by physically pulling or peeling attached sensors from the supporting substrate. The supporting substrate can thereafter be cleaned and reused, or otherwise discarded. The functional coating layer(s) can be applied either before or after other sensor components are removed from the supporting substrate (e.g., by cutting).

### III. METHODS FOR USING ANALYTE SENSOR APPARATUS OF THE INVENTION

Related aspects of the disclosure is a method of sensing an analyte within the body of a mammal, the method comprising implanting an analyte sensor embodiment disclosed herein in to the mammal and then sensing one or more electrical fluctuations such as alteration in current at the working electrode and correlating the alteration in current with the presence of the analyte, so that the analyte is sensed. In one such method, the analyte sensor apparatus senses glucose in the mammal. In an alternative method, the analyte sensor apparatus senses lactate, potassium, calcium, oxygen, pH, and/or any physiologically relevant analyte in the mammal.

Certain analyte sensors having the structure discussed above have a number of highly desirable characteristics which allow for a variety of methods for sensing analytes in a mammal. For example in such methods, the analyte sensor apparatus implanted in the mammal functions to sense an analyte within the body of a mammal for more than 1, 2, 3, 4, 5, or 6 months. Typically, the analyte sensor apparatus so implanted in the mammal senses an alteration in current in response to an analyte within 15, 10, 5 or 2 minutes of the analyte contacting the sensor. In such methods, the sensors can be implanted into a variety of locations within the body of the mammal, for example in both vascular and non-vascular spaces.

### IV. KITS AND SENSOR SETS

In another aspect of the disclosure, a kit and/or sensor set, useful for the sensing an analyte as is described above, is provided. The kit and/or sensor set typically comprises a container, a label and an analyte sensor as described above. Suitable containers include, for example, an easy to open package made from a material such as a metal foil, bottles, vials, syringes, and test tubes. The containers may be formed from a variety of materials such as metals (e.g. foils) paper products, glass or plastic. The label on, or associated with, the container indicates that the sensor is used for assaying the analyte of choice. In some aspects, the container holds a porous matrix that is coated with a layer of an enzyme such as glucose oxidase. The kit and/or sensor set may further include other materials desirable from a commercial and user standpoint, including elements or devices designed to facilitate the introduction of the sensor into the analyte environment, other buffers, diluents, filters, needles, syringes, and package inserts with instructions for use.

### EXAMPLES

### EXAMPLE 1: MECHANISTIC PRINCIPLES AND ASSOCIATED METHODS AND MATERIALS USEFUL FOR PRACTICING EMBODIMENTS OF THE INVENTION

Eliminating sensor signal interference that results from small neutral compounds such as acetaminophen (Mw = 151.17 Daltons) is challenging considering their typically smaller size than sensed analytes such as glucose (Mw = 180.15 Daltons). While the use of polymer(s) thin films in sensors has been attempted in the art, eliminating interference remains problematical because of such film's insufficient density (even if using a very high molecular weight polymer). In order to keep sensor signals at a high level and quick start-up properties, membrane structures used for interference rejection should be extremely dense and hydrophilic.

As disclosed herein, a direct thin film coating followed by a crosslinking of hydrophilic polymer system is an ideal option when considering the requirements listed above.

### IRMS USEFUL IN ANALYTE SENSORS

In typical sensor designs, glucose reaches the enzyme glucose oxidase, where it is oxidized to gluconic acid by oxygen, leaving H₂O₂. One molecule of glucose gives one molecule of H₂O₂ at the expense of one molecule of oxygen:

C₆H₁₂O₆ + H₂O + O₂ → C₅H₁₁O₅COOH + H₂O₂ (1)

At the working electrode H₂O₂ is oxidized and the oxygen is regenerated:

H₂O₂ → O2 + 2H+ + 2e- (2)

It is seen that in case all the enzymatically generated H₂O₂ (Mw = 34.02 Daltons). reaches the working electrode the oxygen is fully recovered.

At the counter electrode some reduction process occurs, the following 3 reactions are possible:

2H2O + 2e- → H2 + 2OH- (3)

H₂O₂ + 2e- → 2OH- (4)

½O2 + 2H+ + 2e- → H2O (5)

Reaction (3) will then proceed as the sensor is in an aqueous environment. Any of the three reactions on the counter electrode will serve to neutralize the protons generated in reaction (2), so the total increase in acidity is caused by the gluconic acid only. The net reaction as sensor functions produces gluconic acid and H2 (at the expense of glucose and H2O.

In embodiments of the invention, the IRM retards diffusion of interfering, electroactive compounds such as acetaminophen and ascorbic acid relative to the diffusion of the H₂O₂ generated by the enzyme reaction, thereby inhibiting and/or eliminating the co-measurements of these interferents because interferent compounds can no longer reach the working electrode during the current measuring period (are inhibited from reaching the working electrode).

Another technical advantage to using sensors having the hydrophilic interference rejection membranes disclosed herein is a reduction of the start-up/initialization time, that is the time after the sensor's placement in vivo that it takes for the sensor to functionally acclimate to this in vivo environments so as to give meaningful readings. Without being bound by a specific scientific theory, it is believed that this is due to the fact that by disposing the IRM directly on the surface of the working electrode, this coverage of the working electrode surface area by the IRM functions to effectively lower the background current much faster than occurs in sensors made without an IRM. In view of this, the initial current overshooting time associated with certain sensor embodiments disappears.

In certain embodiments of the invention the IRM comprises a methacrylate polymer and is produced using silane condensation and silane crosslinking reactions with pHEMA to create a dense membrane structure. As noted above, such semi-permeable membranes function via size exclusion of interfering compounds (e.g. as a sort of molecular sieve). In certain embodiments of the invention, the IRM inhibits the ability of molecules with molecular weight above 140 Daltons to diffuse to the working electrode of an analyte sensor.

In this context, a silane polymerized film can be formed by coating and subjecting to heat treatment of an aqueous solution of an alkoxy silicone on the surface of the electrode consisting of platinum and the enzyme film is formed thereon. A dipodal alkoxy silicone one can use in embodiments of the invention is expressed by the general formula xn-si2[(or)4-n]2 (n=1, 2, 3), where x is functional group such as amino group, epoxy group or thionyl group, r is an alkyl group, or is preferably a hydrolyzable group such as methoxy group or ethoxy group. Such alkoxy compounds form a silanol group by hydrolysis when mixed with water and causes a dehydration polymerization and crosslinking within hydroxyl functional polymer matrix (e.g. a polymer having -OH moieties) when heated. The film consisting of enzyme and film forming material such as albumin or glutaraldehyde can then be used as the enzyme film.

Some embodiments of the invention use silane compounds that contain three inorganic reactive groups on silicon bond well to the metal hydroxyl groups which was formed during a plasma treatment. The alkoxy groups on silicon hydrolyze to silanols through the addition of water. Then the silanols coordinate with metal hydroxyl groups on the inorganic surface to form an oxane bond and eliminate water.

In some crosslinking reactions used to make embodiments of the invention, alkxoy groups on silicon can react with hydoxy groups of polymer pHEMA to couple with each other. For example, in some embodiments of the invention, pHEMAs can be linked by dipodal silane crosslinking agents in order to generate a tightly connected polymer network. Alternatively artisans can use another crosslinking mechanism, for example one based on the condensation with the addition of H2O, the attached -OH group later on came from pHEMA instead of from the substrate metal surface which still realize the pHEMA further crosslinking. Silane is a typical crosslinker suitable for use with a polymer comprising hydroxyl moieties. Urea is another crosslinker suitable for use with hydoxy group of the functional acrylic polymers to form an integrated dense polymer network.

Illustrative formulations of IRMS are listed in the tables below:

**Table 1: pHEMA formulations with different content**

| **PolyHEMA solution** | | 0.40% | 0.50% | 0.75% | 1.00% | 1.50% | 2.00% |
|---|---|---|---|---|---|---|---|
| PolyHMA (sp² 414 ) | g | 0.4 | 0.5 | 0.75 | 1 | 1.25 | 2 |
| C2H5OH | g | 95 | 95 | 95 | 95 | 95 | 95 |
| H2O | g | 5 | 5 | 5 | 5 | 5 | 5 |

**Table 2: IRM formulations with variable silane content**

| IRM Solution | 100% silane | 75% silane | 50% silane |
|---|---|---|---|
| BIS[3-(TRIETHOXYSILYL) | | | |
| PROPYL]UREA 60% in ethanol | 0.11 | 0.0 | 0.058 |
| PhemA solution g | 2 | | 20 |

In certain embodiments of the invention, an IRM solution can be applied via a spray process. In one example of this, after drying in fume hood, the plates coated with polymers were put in a 175°C oven for 6 minute to bake, and then followed by rinsing in DI water for 5 minutes and then drying them. Sensors having such IRM were then built following standard manufacturing protocols. Finished *in vitro* sensor performances were evaluated at PBS, BTS and SITS levels. To test the IRMs, sensor performance in phosphate buffered saline (PBS) was evaluated. After background current was stabilized 10mg/dL acetaminophen solution was introduced followed by introducing into 0.1mM (3.3ppm) **H₂O₂** solution and Isig was recorded.

Hydrophilic silanes from Gelest can be employed in as illustrative suitable hydrophilic crosslinkers for IRM performance evaluation. Among them is Gelest sib 1828.0 bis[3-(triethoxysilyl) propyl]urea, a compound which exhibits good interference rejection capabilities. In order to improve the wettability, interference rejection capacity and ensure a stable binding of an protein layer comprising glucose oxidase (GOx) on top of it, pHEMA is added into the formulation based on its crosslinking ability and solvent compatibility Preliminary experiments provide evidence that higher pHEMA concentrations create more dense membrane structures which result in lower H₂O₂ signals and better interference rejection. Similarly, lower pHEMA concentrations can increase the Isig to H₂O₂ and interference rejection which can be compensated at higher spray volume to get better interference elimination. Using different pHEMA concentrations can be used to produce different membrane surface hydrophilicity if the silane content is not in excess. Changing spray volumes can also adjust the Isig and interference rejection capability. Sensors with very low pHEMA concentrations showed drifting during test process. Such sensors show very minimum drifting when pHEMA concentration reached 1%.

Different molecular weights of pHEMA were examined at 1% concentration based on 3ulx3 volume spray. Figure 3 shows how different molecular weights of pHEMA can impact Isig and interference rejection capabilities. Data in FIG. 3 indicates that higher molecular weight polymers can produce denser membrane with low H₂O₂ permeability and better interference rejection. Lower molecular weight polymers produced a looser structure which gives a higher H₂O₂ permeability and lower interference rejection. A 300kd pHEMA polymer is one mw polymer that provides suitable properties for both Isig and interference rejection levels.

In studies of silane content in IRMs, an original amount of silane which is 0.115g in 20g pHEMA solution is defined as 100%. Silane content variations from 0% to 100% were examined in IRM formulations with a constant 0.5% pHEMA. A typical spray volume is 5uL x 2. For sensors having no IRM, the interference level to 10mg/dL is about 700nA or higher, and H₂O₂ Isig is about 200nA. From the response curves it can be seen there is limited interference rejection with pHEMA only membrane. The Isig loss to H₂O₂ is very minimum comparing with sensors lacking IRMs. However with a 0.25% silane addition a significant interference rejection can be seen. A satisfactory level of rejection can be reached if 100% silane is applied.

In certain embodiments of the invention, a satisfactory Isig and interference rejection balance can be adjusted within 50-75% silane. Correspondingly, the pHEMA content or the spray volume can be increased depending upon hydrophilicity preferences. Depending upon the application and/or environment in which a sensor is used, one typically endeavors to generate a membrane with enhanced hydration and start up properties yet with a lower silane content. Figure 4A shows sensor responses to variations of silane content in IRM. Adjustment of different component content and spray volumes can be used to modulate interference rejection properties in various sensor embodiments. For example, a process using 5uL x2 of 50% silane showed slightly higher interference, but 7uL x2 of 50% silane showed a lower signal than expected. About 5% of water is typically combined with Ethanol to facilitate dissolution of pHEMA compounds.

A variety of permutations from IRM recipes are contemplated. For example, a number of small molecular weight and high boiling point chemicals which can't chemically interact with the polymer matrix and are compatible with the membrane system have been added into IRM formulation in order to create some porosity after being released into water so as to expedite the start up and enhance Isig level. Glycerol as plasticizer can be added to IRM formulation to reduce cracks and expedite initial hydration and start-up considering its hydroscopic character.

Hydrophilic polymer(s) with primary-amine group that can be entrapped in the IRM cross linked matrix are desirable. Some other more hydroscopic polymer with hydroxyl group such as hydroxypropyl cellulose can be added or be used to replace pHEMA to expedite the initial start up.

A variety of other crosslinker(s) can also be used in embodiments of the invention. For example, urea has been used as a crosslinker for pHEMA matrix. Different baking temperature and times have also been used to create IRMs having tailored properties. For example at 200°C, sensors showed slightly higher interference levels and slightly slow start-up issues. At 175°C for 6 minutes, sensor IRMs exhibited the favorable interference rejection, start-up and background current. At 150°C, such sensors showed slightly higher background current and the favorable linearity and the performance is not significantly different from 175°C. So a temperature range of 150°C to 175°C is shown to be a generally acceptable range (although temperatures outside of this range will nonetheless generate functional interference rejection membranes).

Longer baking time such as 20 minutes did not show any benefit to IRM functionality. 5 minutes and 12 minutes also did not show significant performance differences. So 6 minutes at 175°C is a typical baking time (although times outside of this range will nonetheless generate functional interference rejection membranes). As shown above, IRMs can be baked or cured one or more times. In certain embodiments of the invention, baking after the application of each layer of a multilayer IRM appears to give a lower interference signal and lower H₂O₂ signal correspondingly. This appears to be due to a better cross linking at the interfacial surface after a second baking. Better interference rejection can be reached with more times of spray but less volume each time (so more layers of cross linking resulted in denser membrane structure).

Sensor interference rejection is stable over a number of days of usage which demonstrates how durable thin (0.1 µm-0.2 µm) IRM membrane structures are. Typically, the disclosed IRMs can eliminate about 90% of the interfering signals produced by interfering species as compared to control sensors lacking the IRMs.

Slot coating processes were also used to make the IRMs of the invention. In these embodiments, 4% pHEMA and the same concentration of silane as the spray process described above were chosen after testing the coating capability. 0.5 µm one pass, 2 pass and 1um 1pass were chosen as testing conditions. Scientific Polymers (414) and Monomer-Polymer & Dajac Labs (8899) pHEMA were chosen as typical materials. The performance of sensors slotcoated with IRM can compete with sprayed sensors if regarding the Isig to acetaminophen signal dampening and H₂O₂ signalling. Although all of the sensors possess the basic interference rejection capability, most sensors started up significantly slow during the initial 24 hours.

In embodiments of the invention, the working electrode (WE) plating current density was set a slight lower at 70 µA. The 0.5% pHEMA formulation yielded a much more consistent interference rejection results which demonstrated an appropriate Pt surface roughness contributes to the formation of a good IRM. Experiments in which silane was set at 75% of original amount and 1% pHEMA did not show any drift even under a sever ATS system. Consequently, IRM formulations can create an ideal surface condition for glucose oxidase immobilization.

Other compounds such as hydrophilic polymers with hydroxy or primary amine groups can also be added into IRM formulations in order to make them more hydrophilic.

### EXAMPLE 2: FURTHER METHODS AND MATERIALS FOR PRACTICING EMBODIMENTS OF THE INVENTION USING METHACRYLATE POLYMERS

Many amperometric glucose sensors when exposed to acetaminophen exhibit significant noise and additional reactions which produce sensor readings that in turn give false responses to glucose. In order to negate the effects of acetaminophen on sensors, an IRM layer was added to the sensor.

One method for IRM deposition comprises spray coating with a biodot robot. The IRM formulation used in this embodiment is as follows:

**TABLE 3: ILLUSTRATIVE IRM FORMULATIONS**

| **Chemical** | **Percent Mass** |
|---|---|
| Ethanol | 93.5% |
| DI water | 5% |
| Poly-HEMA | 1% |
| BIS[3-(Triethoxysilyl)propyl]urea, 60% in ethanol | 0.5% |

This IRM solution is coated onto the sensor plate after the electrode plating process and before the enzyme process. If the IRM layer were to be placed above the enzyme layer, glucose could be prevented from diffuse through the IRM due to its molecular size.

Typically, the IRM is deposited using a biodot^{®} system. A second parameter for the IRM process characterization was the number of layers of IRM. After each layer of IRM is deposited, the membrane is cross-linked through a baking cycle of 175° C for 6 minutes.

After the IRM processing, the sensors undergoes standard fabrication protocols. Both the enzyme and adhesion promoter cross-link processes were performed using a glutaraldehyde chemical vapor deposition process where a 10 minute cross-link cycle was performed. The adhesion promoter deposition process was performed using 3% adhesion promoter in ethanol. Sterilized sensors were tested in a sits (sensor in-vitro test system) for performance evaluation. During SITS testing, 1mg/dl of acetaminophen was introduced into the system to test the performance of the IRM layer. The acetaminophen was introduced while the system was at glucose 40mg/dl.

All of the test IRM sensors had less of a response to interfering compounds than the production control sensors (i.e. those lacking IRMs). On average the highest response was 117% with 2 µl/cm and 2 layers, while the lowest response was 5.1% with 3 µl/cm and 3 layers. In contrast, production control sensors on average had a 273.4% response to acetaminophen.

The data provided herein reveals that the IRM layer was effective in reducing the effects of acetaminophen interference in amperometric glucose sensors. The parameter that reduced the response was 3 µl/cm and 3 layers of IRM. The combination of the IRM chemistry and spray process reduced the acetaminophen response from 273% to 5% when the sensors are subjected to 1mg/dl of acetaminophen.

### EXAMPLE 3: METHODS AND MATERIALS FOR PRACTICING EMBODIMENTS OF THE INVENTION USING PRIMARY AMINE-BASED POLYMERS

This example illustrates an IRM embodiment constructed from a crosslinked (by glutaraldehyde vapor) film of primary amine-based polymer, oligomers and monomers.

### EQUIPMENT AND MATERIALS:

BioDot Sprayer
Polylysine Hydrobromide
Sigma-Aldrich P1024 Mw 416,300 (HMW)
Sigma-Aldrich P1399 Mw 163,200
Poly(vinylamine) hydrochloride, [CAS: 26336-38-9 Polysciences 23965] MW 25,000
Polyethylenimine, branched (30% solution in water, MW 70,000)
Highly branched polyamine with high charge density. Liquid polymers. Soluble in water at all molecular weights, also soluble in lower alcohols, glycols, and THF.
Polymers contain primary, secondary, and tertiary amine groups in approximately 25/50/25 ratio
Poly(allylamine hydrochloride), [CAS Number: 71550-12-4, Polysciences, 18378] Polymeric primary amine MW ~60,000
Poly(allylamine) solution average Mw ∼65,000, 20 wt. % in H2O Sigma-Aldrich 479144
Polyethylene oxide), diamine terminated, scientific polymers 817 [CAS 65605-36-9, MW 2000]

### Jeffamine Series primary amine-based polymers

D-4000(XTJ-510)
ED-2003(XTJ-502)
T-3000(XTJ-509)
T-5000
N-(2-aminoethyl)-3-aminopropyltriethoxysilane (3-aminopropyl)triethoxysilane) GELEST SIA0590.5

Eliminating interference in amperometric sensors which is caused by small neutral molecules such as acetaminophen (MW 150 Daltons) is challenging considering that these compounds are typically smaller in size than glucose (MW 180 Daltons). Direct coating of thin film polymer(s) may not eliminate interference perhaps due to insufficient density (even if using a very high molecular weight polymer). In order to keep sensor signal at a high level and quick start-up, membrane structure should typically be dense and hydrophilic.

A direct thin film coating followed by a crosslinking of a hydrophilic polymer system is usually a good choice considering the requirement listed above. Heated silane only condensation technology can be adapted for such processes. Effort has been made here by applying dipodal hydrophilic silane crosslinked with pHEMA good interference rejection has been reached with adjustability based on requirement. Crosslinking of primary amine-based polymer with glutaraldehyde is yet another option to provide a dense and more hydrophilic membrane system instead of heating condensation.

The solvent for polylysine is di water and concentration range from 0.5% to 1.0%. The IRM solution was sprayed based on art accepted procedures followed by static chamber or CVD chamber crosslinking for 2 hours. After crosslinking, the plates were rinsed in di water to get rid of the free deposited glutaraldehyde on the surfaces. Fully sensors were built following standard glucose sensor procedure, AP crosslink and GLM casting.

IRM performance of the polylysine polymers was evaluated. After background current was stabilized, 10mg/dL acetaminophen solution was introduced followed by introducing into 0.1mM (3.3ppm) H₂O₂ solution and Isig was recorded. Fully built sensors were evaluated by introducing into 100mg/dL glucose solution and 1mg/dL acetaminophen added 100mg/dL glucose solution in order to compare the Isig increase due to the acetaminophen.

Figures 5A and 5B show sensor Interference Rejection Performance of IRM with 1% polylysine single crosslinking. This IRM composition performed very efficiently using a single layer applied via spraying. H₂O₂ ISig is not significantly decreased with IRM coating even with 2 layers of spray. Two layers of coating gave a better interference rejection capability. The interference rejection level is equivalent to the current pHEMA-silane system. Figure 5C shows IRM performances after 2 week of PBS testing. Usually sensor interference rejection performance does not show significant change if only use for a week. However, from Figure 5C it does show some difference from different process parameters, 4uLx2 shows the most stable interference capability, all of the H₂O₂ permeability doesn't change significantly. Figure 5D shows IRM performances from different molecular weight of polylysine. Higher MW polylysine shows better interference rejection. With a single layer of spray, the performance stability of this higher MW polylysine is better than its lower molecular weight counter part. Figure 5E shows the performance comparison of polylysine and pHEMA-silane IRM compositions. Figure 5F shows IRM sensor start-up performance. The sensors started up within 30 minutes and did not show significant drift.

In summary, polylysine gives a competitive performance as compared to known IRM materials. Polyallylamine further showed encouraging results. High molecular weight or very high molecular weight polymer with appropriate crosslinking capability provides functional IRM that can be disposed on a rough Pt working electrode surface. In addition, polylysine is compatible with the protein and GOX layers typically used in sensors. Consequently, there should be less of a possibility of cracking or delamination issues with sensors that employ such IRMS.

## Claims

1. An amperometric analyte sensor apparatus (100) comprising:
a base layer (102);
a conductive layer (104) disposed on the base layer (102) and comprising a working electrode;
an interference rejection membrane disposed on an electroactive surface of the working electrode **characterised in that** the interference rejection membrane comprises crosslinked primary amine polymers or crosslinked methacrylate polymers; and
an analyte sensing layer (110).

2. The analyte sensor apparatus of claim 1, wherein the crosslinked methacrylate polymers comprise Poly(2-hydroxyethyl methacrylate) polymers having an average molecular weight between 100 and 1000 kilodaltons.

3. The analyte sensor apparatus of claim 1, wherein the crosslinked primary amine polymers comprise:
polylysine polymers;
poly(allylamine) polymers;
amine terminated polyethylene oxide) polymers;
poly(vinylamine) polymers; or
polyethylenimine polymers;
having an average molecular weight between 4 and 500 kilodaltons.

4. The analyte sensor apparatus of claim 1, wherein the interference rejection membrane is between 0.1 µm and 1.0 µm thick.

5. The analyte sensor apparatus of claim 1, wherein the interference rejection membrane inhibits the diffusion therethrough of compounds having a molecular weight greater than 140 Daltons.

6. The analyte sensor apparatus of claim 1, wherein the interference rejection membrane inhibits the diffusion of acetaminophen therethrough in a manner that decreases a signal in the analyte sensor apparatus that results from a concentration of acetaminophen by at least 50% as compared to a control analyte sensor apparatus lacking the interference rejection membrane.

7. The analyte sensor apparatus of claim 1, wherein the hydrophilic crosslinking agent comprises:
a urea;
a hydrophilic organofunctional dipodal alkoxysilane; or
glutaraldehyde.

8. The analyte sensor apparatus of claim 1, wherein an electrochemically reactive surface of the working electrode comprises platinum black having an irregular surface structure.

9. The analyte sensor apparatus of claim 1, wherein:
the interference rejection membrane is in direct contact with an electrochemically reactive surface of the working electrode; and
the analyte sensing layer is in direct contact with the interference rejection membrane.

10. The analyte sensor apparatus of claim 1, wherein the analyte sensor apparatus is implantable within a mammal.

11. The analyte sensor apparatus of claim 1, further comprising at least one of:
a protein layer disposed on the analyte sensing layer;
an analyte modulating layer disposed on the analyte sensing layer of the protein layer, wherein the analyte modulating layer comprises a composition that modulates the diffusion of an analyte diffusing through the analyte modulating layer;
an adhesion promoting layer disposed on the analyte sensing layer, wherein the adhesion promoting layer promotes the adhesion between the analyte sensing layer and an analyte modulating layer; or
a cover layer disposed on the analyte sensor apparatus, wherein the cover layer comprises an aperture positioned on the cover layer so as to facilitate an analyte present in the mammal contacting and diffusing through an analyte modulating layer; and contacting the analyte sensing layer.

12. The analyte sensor apparatus of claim 1, wherein the conductive layer comprises a plurality of electrodes including the working electrode, a counter electrode and a reference electrode.

13. The analyte sensor of claim 12, wherein the conductive layer comprises a plurality of working electrodes, counter electrodes and reference electrodes; and
the plurality of working, counter and reference electrodes are grouped together as a unit and positionally distributed on the conductive layer in a repeating pattern of units.

14. The analyte sensor apparatus of claim 1, wherein the analyte sensing layer comprises an oxidoreductase that generates hydrogen peroxide upon exposure to a substrate for the oxidoreductase, wherein the amount of hydrogen peroxide generated by the oxidoreductase is proportional to the amount of substrate exposed to the oxidoreductase.

15. The analyte sensor of claim 1, wherein a pulsed voltage is used to obtain a signal from an electrode.

## Patentansprüche

1. Amperometrische Analytsensoreinrichtung (100), umfassend:
eine Basisschicht (102);
eine leitfähige Schicht (104), die auf der Basisschicht (102) angeordnet ist und umfassend eine Arbeitselektrode;
eine Störunterdrückungsmembran, die auf einer elektroaktiven Oberfläche der Arbeitselektrode angeordnet ist, **dadurch gekennzeichnet, dass** die Störunterdrückungsmembran vernetzte Primäraminpolymere oder vernetzte Methacrylatpolymere umfasst; und
eine Analytsensorschicht (110);

2. Analytsensoreinrichtung nach Anspruch 1, wobei die vernetzten Methacrylatpolymere Poly(2-hydroxyethylmethacrylat)polymere, die ein durchschnittliches Molekulargewicht von zwischen 100 und 1000 Kilodalton aufweisen, umfassen.

3. Analytsensoreinrichtung nach Anspruch 1, wobei die vernetzten Primäraminpolymere umfassen:
Polylysinpolymere;
Poly(allylamin)polymere;
aminterminierte Poly(ethylenoxid)polymere;
Poly(vinylamin)polymere; oder
Polyethyleniminpolymere,
die ein durchschnittliche Molekulargewicht von zwischen 4 und 500 Kilodalton aufweisen.

4. Analytsensoreinrichtung nach Anspruch 1, wobei die Störunterdrückungsmembran zwischen 0,1 µm und 1,0 µm dick ist.

5. Analytsensoreinrichtung nach Anspruch 1, wobei die Störunterdrückungsmembran die Diffusion dahindurch von Verbindungen, die ein Molekulargewicht von über 140 Dalton aufweisen, hemmt.

6. Analytsensoreinrichtung nach Anspruch 1, wobei die Störunterdrückungsmembran die Diffusion von Acetaminophen dahindurch in einer Weise hemmt, die ein Signal in der Analytsensoreinrichtung, das aus einer Konzentration von Acetaminophen resultiert, um mindestens 50 %, verglichen mit einer Kontroll-Analytsensoreinrichtung ohne die Störunterdrückungsmembran, verringert.

7. Analytsensoreinrichtung nach Anspruch 1, wobei das hydrophile Vernetzungsmittel umfasst:
einen Harnstoff;
ein hydrophiles organofunktionelles dipodales Alkoxysilan; oder
Glutaraldehyd.

8. Analytsensoreinrichtung nach Anspruch 1, wobei eine elektrochemisch reaktive Oberfläche der Arbeitselektrode Platinschwarz, das eine unregelmäßige Oberflächenstruktur aufweist, umfasst.

9. Analytsensoreinrichtung nach Anspruch 1, wobei:
die Störunterdrückungsmembran in direktem Kontakt mit einer elektrochemisch reaktiven Oberfläche der Arbeitselektrode ist; und
die Analytsensorschicht in direktem Kontakt mit der Störunterdrückungsmembran ist.

10. Analytsensoreinrichtung nach Anspruch 1, wobei die Analytsensoreinrichtung innerhalb eines Säugetiers implantierbar ist.

11. Analytsensoreinrichtung nach Anspruch 1, ferner umfassend mindestens eines von:
einer Proteinschicht, die auf der Analytsensorschicht angeordnet ist;
einer Analytmodulationsschicht, die auf der Analytsensorschicht oder der Proteinschicht angeordnet ist, wobei die Analytmodulationsschicht eine Zusammensetzung umfasst, die die Diffusion eines Analyts, der durch die Analytmodulationsschicht diffundiert, moduliert;
einer Haftvermittlerschicht, die auf der Analytsensorschicht angeordnet ist, wobei die Haftvermittlerschicht die Haftung zwischen der Analytsensorschicht und einer Analytmodulationsschicht vermittelt; oder
einer Deckschicht, die auf der Analytsensoreinrichtung angeordnet ist, wobei die Deckschicht eine Öffnung, die auf der Deckschicht positioniert ist, umfasst, um zu ermöglichen, dass ein Analyt, der in dem Säugetier vorhanden ist, eine Analytmodulationsschicht kontaktiert und durch diese diffundiert; und Kontaktieren der Analytsensorschicht.

12. Analytsensoreinrichtung nach Anspruch 1, wobei die leitfähige Schicht eine Vielzahl von Elektroden, einschließlich der Arbeitselektrode, einer Gegenelektrode und einer Bezugselektrode, umfasst.

13. Analytsensor nach Anspruch 12, wobei die leitfähige Schicht eine Vielzahl von Arbeitselektroden, Gegenelektroden und Bezugselektroden umfasst; und
die Vielzahl der Arbeits-, Gegen- und Bezugselektroden zu einer Einheit zusammen gruppiert und in einem sich wiederholenden Muster von Einheiten auf der leitfähigen Schicht positionell verteilt werden.

14. Analytsensoreinrichtung nach Anspruch 1, wobei die Analytsensorschicht eine Oxidoreduktase, die bei Exposition mit einem Substrat für die Oxidoreduktase Wasserstoffperoxid erzeugt, umfasst, wobei die Menge des Wasserstoffperoxids, das durch die Oxidoreduktase erzeugt wird, zu der Menge des Substrats, die der Oxidoreduktase exponiert ist, proportional ist.

15. Analytsensor nach Anspruch 1, wobei eine gepulste Spannung verwendet wird, um ein Signal von einer Elektrode zu erhalten.

## Revendications

1. Appareil capteur ampérométrique d'analyte (100) comprenant :
une couche de base (102) ;
une couche conductrice (104) disposée sur la couche de base (102) et comprenant une électrode de travail ;
une membrane de rejet des interférences disposée sur une surface électroactive de l'électrode de travail, **caractérisé en ce que** la membrane de rejet des interférences comprend des polymères d'amine primaire réticulés ou des polymères de méthacrylate réticulés ; et
une couche de détection d'analyte (110).

2. Appareil capteur d'analyte selon la revendication 1, dans lequel les polymères de méthacrylate réticulés comprennent des polymères de poly(méthacrylate de 2-hydroxyéthyle) ayant un poids moléculaire moyen compris entre 100 et 1000 kilodaltons.

3. Appareil capteur d'analyte selon la revendication 1, dans lequel les polymères d'amine primaire réticulés comprennent :
des polymères de polylysine ;
des polymères de poly(allylamine) ;
des polymères de poly(oxyde d'éthylène) terminés par une amine ;
des polymères de poly(vinylamine) ; ou
des polymères de polyéthylèneimine ;
ayant un poids moléculaire moyen compris entre 4 et 500 kilodaltons.

4. Appareil capteur d'analyte selon la revendication 1, dans lequel la membrane de rejet des interférences a une épaisseur comprise entre 0,1 µm et 1,0 µm.

5. Appareil capteur d'analyte selon la revendication 1, dans lequel la membrane de rejet des interférences inhibe la diffusion à travers elle de composés ayant un poids moléculaire supérieur à 140 daltons.

6. Appareil capteur d'analyte selon la revendication 1, dans lequel la membrane de rejet des interférences inhibe la diffusion de l'acétaminophène à travers elle d'une manière qui diminue un signal dans l'appareil capteur d'analyte qui résulte d'une concentration d'acétaminophène d'au moins 50 % comparé à un appareil capteur d'analyte de commande manquant de membrane de rejet des interférences.

7. Appareil capteur d'analyte selon la revendication 1, dans lequel l'agent de réticulation hydrophile comprend :
une urée ;
un alcoxysilane dipodal organofonctionnel hydrophile ; ou
glutaraldéhyde.

8. Appareil capteur d'analyte selon la revendication 1, dans lequel une surface électrochimiquement réactive de l'électrode de travail comprend du noir de platine ayant une structure de surface irrégulière.

9. Appareil capteur d'analyte selon la revendication 1, dans lequel :
la membrane de rejet des interférences est en contact direct avec une surface électrochimiquement réactive de l'électrode de travail ; et
la couche de détection d'analyte est en contact direct avec la membrane de rejet des interférences.

10. Appareil capteur d'analyte selon la revendication 1, dans lequel l'appareil capteur d'analyte est implantable dans un mammifère.

11. Système capteur d'analyte selon la revendication 1, comprenant en outre au moins l'une parmi :
une couche de protéines disposée sur la couche de détection d'analyte ;
une couche de modulation d'analyte disposée sur la couche de détection d'analyte ou la couche de protéines, dans lequel la couche de modulation d'analyte comprend une composition qui module la diffusion d'un analyte diffusant à travers la couche de modulation d'analyte ;
une couche de promotion d'adhérence disposée sur la couche de détection d'analyte, dans lequel la couche de promotion d'adhérence promeut l'adhésion entre la couche de détection d'analyte et une couche de modulation d'analyte ; ou
une couche de couverture disposée sur l'appareil capteur d'analyte, dans lequel la couche de couverture comprend une ouverture positionnée sur la couche de couverture de façon à permettre à un analyte présent dans le mammifère de venir en contact et de diffuser à travers une couche de modulation d'analyte ; et de venir en contact avec la couche de détection d'analyte.

12. Appareil capteur d'analyte selon la revendication 1, dans lequel la couche conductrice comprend une pluralité d'électrodes comportant l'électrode de travail, une contre-électrode et une électrode de référence.

13. Capteur d'analyte selon la revendication 12, dans lequel la couche conductrice comprend une pluralité d'électrodes de travail, de contre-électrodes et d'électrodes de référence ; et
la pluralité d'électrodes de travail, de contre-électrodes et d'électrodes de référence sont regroupées en une unité et réparties positionnellement sur la couche conductrice dans un motif répétitif d'unités.

14. Appareil capteur d'analyte selon la revendication 1, dans lequel la couche de détection d'analyte comprend une oxydoréductase qui génère du peroxyde d'hydrogène lors de l'exposition à un substrat pour l'oxydoréductase, dans lequel la quantité de peroxyde d'hydrogène générée par l'oxydoréductase est proportionnelle à la quantité de substrat exposée à l'oxydoréductase.

15. Capteur d'analyte selon la revendication 1, dans lequel une tension pulsée est utilisée pour obtenir un signal à partir d'une électrode.
